(19) Europäisches Patentamt / European Patent Office / Office européen des brevets

(11) EP 2 490 991 B1

(12) # EUROPÄISCHE PATENTSCHRIFT

(45) Veröffentlichungstag und Bekanntmachung des Hinweises auf die Patenterteilung:
**20.11.2013 Patentblatt 2013/47**

(21) Anmeldenummer: **10760617.0**

(22) Anmeldetag: **01.10.2010**

(51) Int Cl.:
***C07C 15/58*** *(2006.01)* ***C07C 15/60*** *(2006.01)*
***C09K 19/18*** *(2006.01)*

(86) Internationale Anmeldenummer:
**PCT/EP2010/006014**

(87) Internationale Veröffentlichungsnummer:
**WO 2011/047781 (28.04.2011 Gazette 2011/17)**

(54) **VERBINDUNGEN FÜR EIN FLÜSSIGKRISTALLINES MEDIUM UND VERWENDUNG FÜR HOCHFREQUENZBAUTEILE**

COMPOUNDS FOR A LIQUID CRYSTAL MEDIUM AND USE FOR HIGH-FREQUENCY COMPONENTS

COMPOSÉS POUR UN MILIEU DE CRISTAUX LIQUIDES ET UTILISATION POUR COMPOSANTS À HAUTE FRÉQUENCE

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **24.10.2009 DE 102009050632**

(43) Veröffentlichungstag der Anmeldung:
**29.08.2012 Patentblatt 2012/35**

(73) Patentinhaber: **Merck Patent GmbH**
**64293 Darmstadt (DE)**

(72) Erfinder:
- **JASPER, Christian**
  **64283 Darmstadt (DE)**
- **MONTENEGRO, Elvira**
  **69469 Weinheim (DE)**
- **PAULUTH, Detlef**
  **64372 Ober-Ramstadt (DE)**
- **REIFFENRATH, Volker**
  **64380 Roßdorf (DE)**
- **MANABE, Atsutaka**
  **64625 Bensheim (DE)**

(74) Vertreter: **Derow, Stephan et al**
**C/o Merck Patent GmbH**
**64271 Darmstadt (DE)**

(56) Entgegenhaltungen:
- **WU S-T ET AL: "HIGH BIREFRINGENCE AND WIDE NEMATIC RANGE BIS-TOLANE LIQUID CRYSTALS" APPLIED PHYSICS LETTERS, AIP, AMERICAN INSTITUTE OF PHYSICS, MELVILLE, NY, US LNKD- DOI:10.1063/1.123066, Bd. 74, Nr. 3, 18. Januar 1999 (1999-01-18), Seiten 344-346, XP000805566 ISSN: 0003-6951**
- **GAUZA ET AL: "Super high birefringence isothiocyanato biphenyl-biolane liquid crystals" JAPANESE JOURNAL OF APPLIED PHYSICS, Bd. 43, Nr. 11A, 1. Januar 2004 (2004-01-01), Seiten 7634-7638, XP002609353 Japan Society of Applied Physics ISSN: 0021-4922**
- **YATABE ET AL: "Liquid crystalline conjugated oligomers: synthesis and mesomorphic properties of laterally and terminally alkyl-substituted oligo(1,4-phenyleneethynylene)s" JOURNAL OF MATERIALS CHEMISTRY., Bd. 18, 1. Januar 2008 (2008-01-01), Seiten 4468-4477, XP002609354 THE ROYAL SOCIETY OF CHEMISTRY, CAMBRIDGE. ISSN: 0959-9428**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 490 991 B1

## Beschreibung

Die vorliegende Erfindung betrifft neue chemische Verbindungen mit zwei oder mehr C-C-Dreifachbindungen und mindestens einem 1,4-Naphthylenrest oder einem 1,4-Anthracenylenrest, damit zusammengestellte flüssigkristalline Medien und diese Medien enthaltende Hochfrequenzbauteile, insbesondere Antennen, speziell für den Gigahertzbereich. Die flüssigkristallinen Medien dienen beispielsweise zur Phasenschiebung von Mikrowellen für abstimmbare "phased-array" Antennen oder für abstimmbare Zellen von Mikrowellenantennen basierend auf "reflectarrays".

Flüssigkristalline Medien werden seit längerem in elektrooptischen Anzeigen (Liquid Crystal Displays - LCDs) genutzt, um Informationen anzuzeigen.

In neuerer Zeit werden flüssigkristalline Medien jedoch auch für die Verwendung in Komponenten, bzw. in Bauteilen, für die Mikrowellentechnik vorgeschlagen, wie z.B. in DE 10 2004 029 429 A und in JP 2005-120208 (A).

Eine technisch wertvolle Anwendung der flüssigkristallinen Medien in der Hochfrequenztechnik beruht auf ihrer Eigenschaft, dass sie sich durch eine variable Spannung in ihren dielektrischen Eigenschaften steuern lassen, besonders für den Gigahertzbereich. Somit lassen sich abstimmbare Antennen konstruieren, die keine beweglichen Teile beinhalten (A. Gaebler, A. Moessinger, F. Goelden, et al., "Liquid Crystal-Reconfigurable Antenna Concepts for Space Applications at Microwave and Millimeter Waves," International Journal of Antennas and Propagation, vol. 2009, Article ID 876989, 7 pages, 2009. doi:10.1155/2009/876989).

A. Penirschke, S. Müller, P. Scheele, C. Weil, M. Wittek, C. Hock und R. Jakoby: "Cavity Perturbation Method for Characterization of Liquid Crystals up to 35GHz", 34th European Microwave Conference -Amsterdam, S. 545-548 beschreibt unter anderen die Eigenschaften der bekannten, flüssigkristallinen Einzelsubstanz K15 (Merck KGaA, Deutschland) bei einer Frequenz von 9 GHz.

1-(Phenylethinyl)tolane, nachfolgend auch Bistolanverbindungen genannt, sind dem Fachmann bekannt. Z.B. offenbart Wu, S.-T., Hsu, C.-S., Shyu, K.-F., Appl. Phys. Lett., 74 (3), (1999), 344-346 verschiedene flüssigkristalline Bistolanverbindungen mit einer lateralen Methylgruppe der Formel

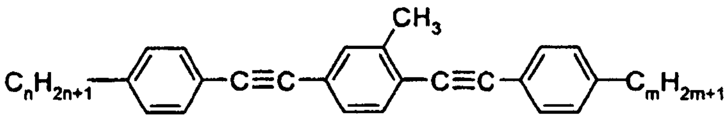

Gauza et al., Jap. J. Appl. Phys. (2004), 11A, 7634-7638 offenbart Bistolane mit einer terminalen Isothiocyanat-Gruppe.

Eine Verbindung der Formel

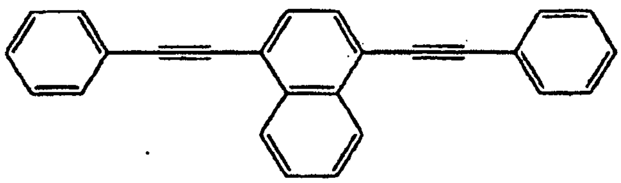

oder abgeleitete Derivate sind beschrieben als Bestandteile von organischen Dünnschicht-Transistoren (EP 2 073 290 A1), als photosensibilisierende Farbstoffe zur Steuerung eines "photoacid generating systems" (WO 2008/021208 A2) und als Bestandteile von Datenaufzeichnungsmedien (JP 2004-082439 A). Flüssigkristalline Eigenschaften und ihre Verwendung in flüssigkristallinen Medien sind bisher nicht beschrieben.

DE 10 2004 029 429 A beschreibt die Anwendung von herkömmlichen Flüssigkristallmedien in der Mikrowellentechnik, unter anderem in Phasenschiebern. Dort werden bereits flüssigkristalline Medien bezüglich ihrer Eigenschaften im entsprechenden Frequenzbereich untersucht.

Die bisher bekannten Zusammensetzungen oder Einzelverbindungen sind jedoch in der Regel mit Nachteilen behaftet. Die meisten von ihnen führen, neben anderen Mängeln, zu unvorteilhaft hohen Verlusten und/oder unzureichenden Phasenverschiebungen bzw. zu geringer Materialgüte.

Für die Anwendung in der Hochfrequenztechnik werden flüssigkristalline Medien mit besonderen, bislang eher ungewöhnlichen, ungebräuchlichen Eigenschaften, bzw. Kombinationen von Eigenschaften benötigt.

Somit sind neue Komponenten für flüssigkristalline Medien mit verbesserten Eigenschaften erforderlich. Insbesondere müssen der Verlust im Mikrowellenbereich verringert und die Materialgüte ($\eta$) verbessert werden.

Außerdem besteht der Bedarf das Tieftemperaturverhalten der Bauteile zu verbessern. Hier sind sowohl eine Verbesserung der Betriebseigenschaften, wie auch der Lagerfähigkeit nötig.

Es besteht daher ein erheblicher Bedarf an flüssigkristallinen Medien mit geeigneten Eigenschaften für entsprechende praktische Anwendungen.

Überraschenderweise wurde nun gefunden, dass mit den erfindungsgemäßen Verbindungen flüssigkristalline Medien mit einem geeigneten, nematischen Phasenbereich und hohem $\Delta n$ verwirklicht werden können, welche die Nachteile der Materialien des Standes der Technik nicht oder zumindest nur in erheblich geringerem Maße aufweisen.

Die Erfindung betrifft Verbindungen der Formel I,

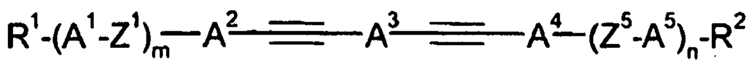

I

worin

$A^{1-5}$ unabhängig voneinander

a) einen Rest der Formeln

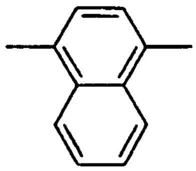

oder

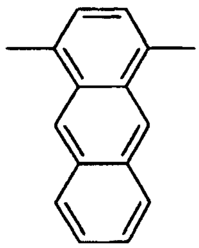

,

b) 1,4-Phenylen, worin ein oder mehrere, bevorzugt ein bis zwei CH-Gruppen durch N ersetzt sein können,

c) trans-1,4-Cyclohexylen oder Cyclohexenylen, worin auch eine oder zwei nicht benachbarte $CH_2$-Gruppen durch -O-und/oder -S- ersetzt sein können, und worin H durch F ersetzt sein kann,

oder

d) einen Rest aus der Gruppe 1,4-Bicyclo(2,2,2)-octylen, Cylcobut-1,3-diyl, Spiro[3.3]heptan-2,6-diyl, Thiophen-2,5-diyl, Thiophen-2,4-diyl, Furan-2,5-diyl, Furan-2,4-diyl,

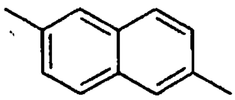

oder

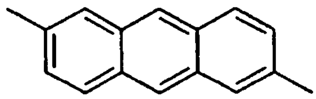

,

und worin in den Gruppen a), b), c) und d)

auch ein oder mehrere H-Atome gegen Br, Cl, F, CN, -NCS, -SCN, $SF_5$, $C_1$-$C_{10}$ Alkyl, $C_1$-$C_{10}$ Alkoxy oder eine ein- oder mehrfach fluorierte $C_1$-$C_{10}$ Alkyl- oder Alkoxygruppe ersetzt sein können,

und wobei

mindestens ein Rest aus $A^1$ bis $A^5$, bevorzugt aus $A^2$, $A^3$ und $A^4$, einen Rest nach a) darstellt,

$R^1$ und $R^2$ jeweils unabhängig voneinander einen halogenierten oder unsubstituierten Alkylrest mit 1 bis 15 C-Atomen, wobei in diesen Resten auch eine oder mehrere $CH_2$-Gruppen jeweils unabhängig voneinander durch -C≡C-, -CH=CH-, -CF=CF-, -CF=CH-, -CH=CF-, -(CO)O-, -O(CO)-, -(CO)-, -O- oder -S- so ersetzt sein können, dass O- oder S-Atome nicht direkt miteinander verknüpft sind, F, Cl, Br, CN, $CF_3$, $OCF_3$, SCN, NCS oder $SF_5$ bedeutet,

$Z^1$, $Z^5$ unabhängig voneinander, eine Einfachbindung, -C≡C-, -CH=CH-, $-CH_2O-$, -(CO)O-, $-CF_2O-$, $-CF_2CF_2-$, $-CH_2CF_2-$, $-CH_2CH_2-$, $-(CH_2)_4-$, -CH=CF- oder -CF=CF-, wobei asymmetrische Brücken nach beiden Seiten orientiert sein können, und

m, n unabhängig voneinander 0, 1 oder 2

bedeuten,

wobei Verbindungen der Formel I-X-1,

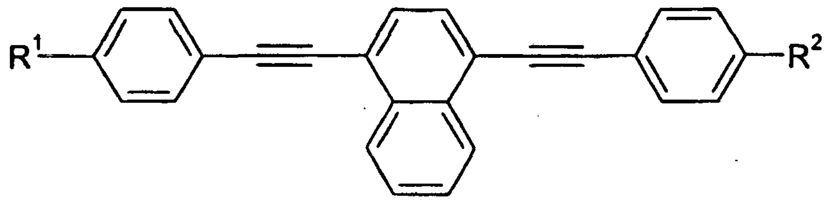

I-X-1

worin
$R^1$ und $R^2$ jeweils gleichzeitig $CH_3$, n-$C_6H_{11}$, $CF_3$, F oder $OCH_3$ sind, oder $R^1$ ein tert-Butyl und $R^2$ ein -CN bedeuten, und die Verbindung der Formel I-X-2

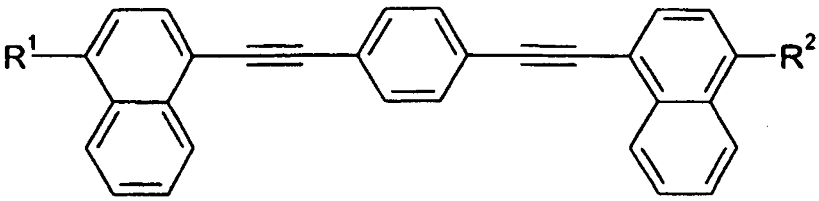

I-X-2

worin
$R^1$ und $R^2$ gleichzeitig n-$C_4H_9$ bedeuten,
ausgeschlossen sind.

Die Verbindungen der Formel I-X-1 sind in der Druckschrift EP 2073290 A1, WO 2008/021208 oder JP 2004-82439 A, die der Formel I-X-2 in der JP 2004-82439 A offenbart.

Die erfindungsgemäßen Verbindungen besitzen einen hohen Klärpunkt, eine außerordentlich hohe optische Anisotropie (An), sowie eine vorteilhafte hohe Rotationsviskosität. Sie besitzen allein oder in Mischung mit weiteren mesogenen Komponenten über einen breiten Temperaturbereich eine nematische Phase. Diese Eigenschaften machen sie besonders geeignet für die Verwendung in Bauteilen für die Hochfrequenztechnik, insbesondere in flüssigkristallinen Phasenschiebern.

Bevorzugt stellen einer oder zwei der Rest aus $A^2$, $A^3$ und $A^4$ einen optional substituierten Rest nach Definition a) der Reste dar, besonders bevorzugt einer. Besonders bevorzugt ist mindestens der Rest $A^3$ ein Rest nach Definition a). Unter den Resten aus der Gruppe a) ist besonders bevorzugt der 1,4-Naphthylenrest.

Der Zähler m ist bevorzugt 0 oder 1, besonders bevorzugt 0. Der Zähler n ist bevorzugt 0 oder 1, besonders bevorzugt 0. Die Summe aus m und n ist bevorzugt 0 oder 1.

Die Ringgruppen $A^1$ und $A^5$ sind bevorzugt ein 1,4-Phenylen, worin auch ein oder mehrere H-Atome gegen Br, Cl, F, CN, Alkyl ($C_1$-$C_{10}$), Methoxy oder eine ein- oder mehrfach fluorierte Methyl- oder Methoxygruppe ersetzt sein können.

Die Brückengruppen $Z^1$ und $Z^5$ sind bevorzugt eine Einfachbindung, -C≡C- oder -CH=CH-, besonders bevorzugt eine Einfachbindung.

Einer der Reste $R^1$ oder $R^2$, bevorzugt $R^1$, bedeutet bevorzugt einen geradkettigen Alkylrest mit 1 bis 15 C-Atomen, wobei in diesen Resten auch eine oder mehrere $CH_2$-Gruppen jeweils unabhängig voneinander durch -C≡C-, -CH=CH-, -(CO)O-, -O(CO)-, -(CO)- oder -O- so ersetzt sein können, dass O-Atome nicht direkt miteinander verknüpft sind. Besonders bevorzugt bedeutet $R^2$ einen Alkylrest mit 2 bis 5 C-Atomen, wobei in diesem Rest auch eine oder mehrere $CH_2$-Gruppen jeweils unabhängig voneinander durch -C≡C-, -CH=CH-, -CF=CF-, -CF=CH-, -CH=CF-, -(CO)O-, -O(CO)- oder -(CO)- so ersetzt sein können, dass O-Atome nicht direkt miteinander verknüpft sind, F, Cl, Br, $CF_3$, $OCF_3$, SCN, NCS oder $SF_5$. Vorzugsweise nimmt keiner oder nur einer der Reste $R^{1/2}$ eine Bedeutung ausgewählt aus F, Cl, Br, CN, $CF_3$, $OCF_3$, SCN, NCS oder $SF_5$ an.

Bevorzugte Ausführungsformen der Erfindung sind daher ausgewählt aus den folgenden beispielhaften Strukturen:

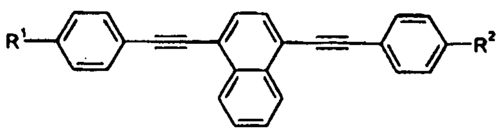

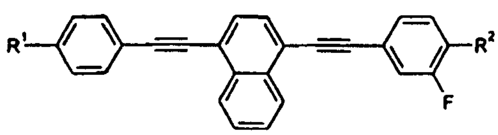

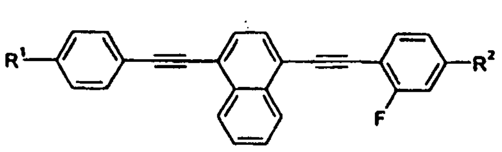
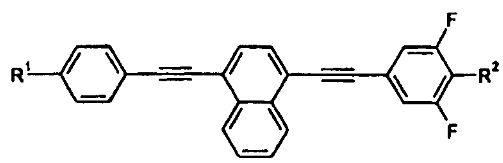

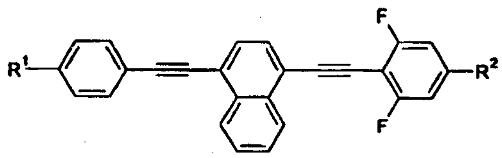
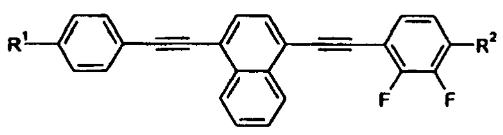

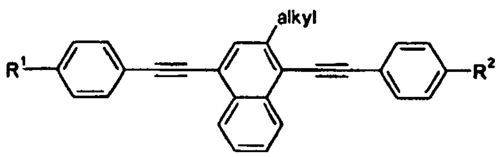
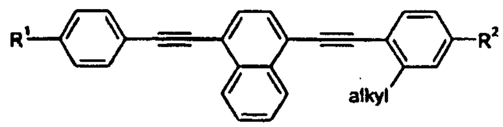

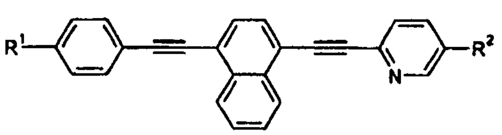
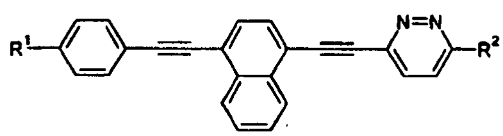

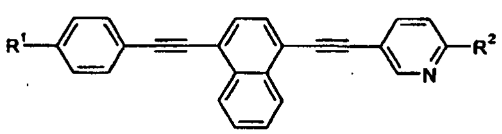
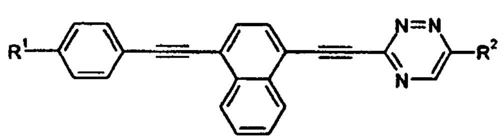

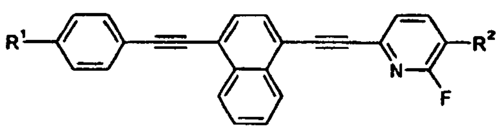
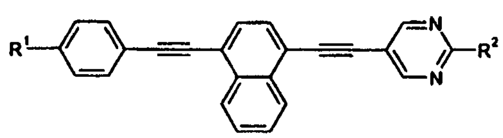

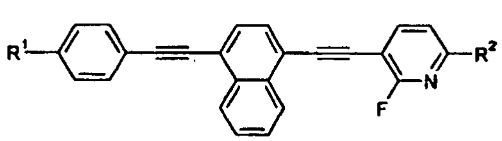
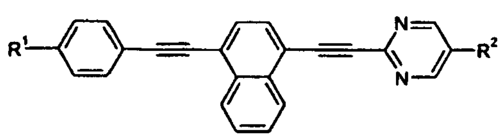

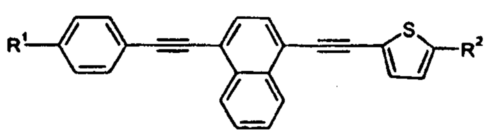

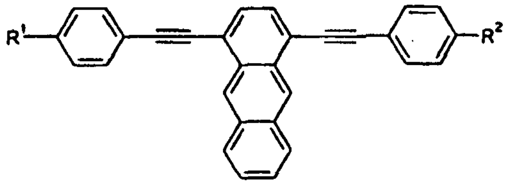

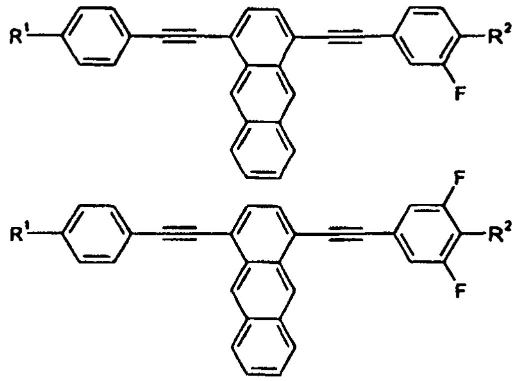

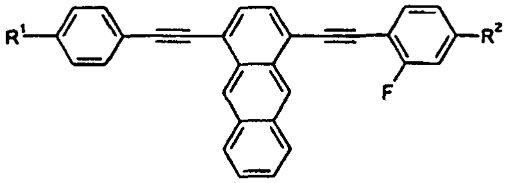

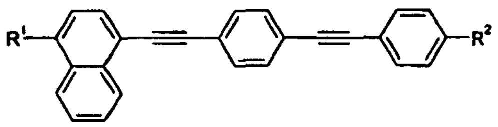

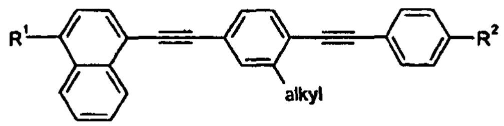

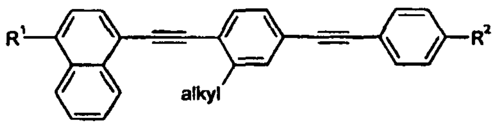

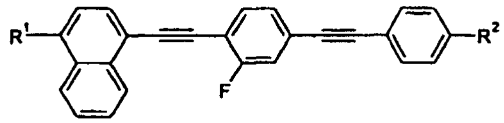

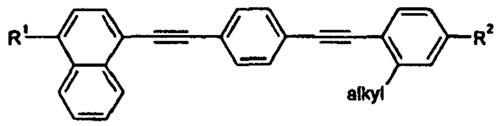

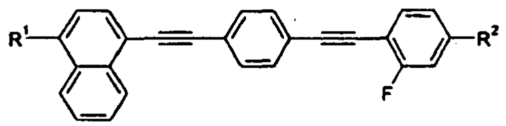

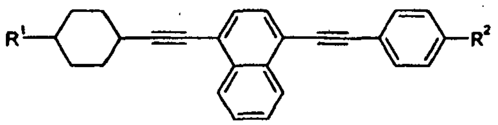

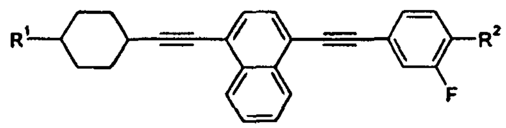

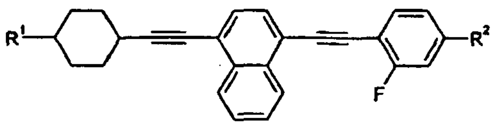

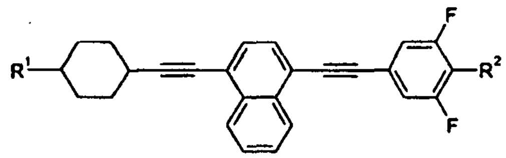

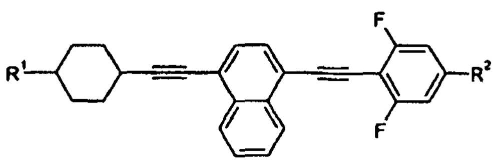

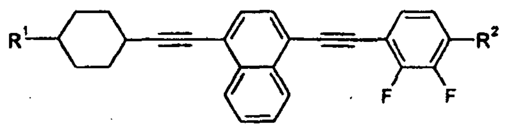

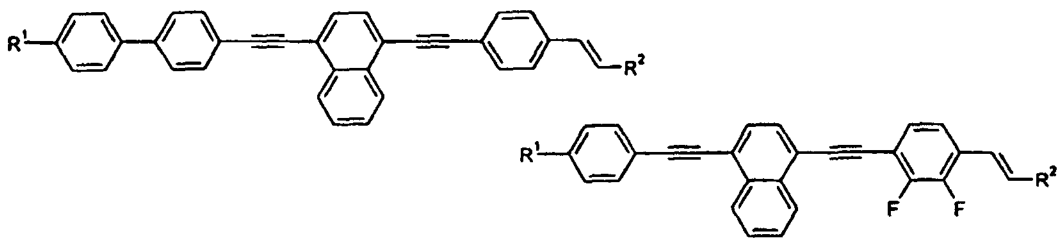

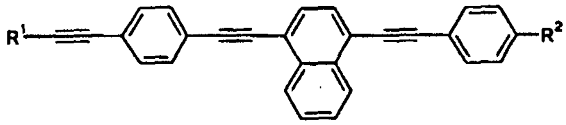

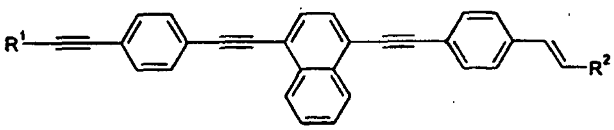

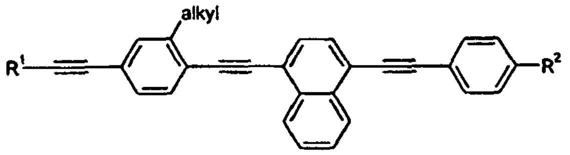

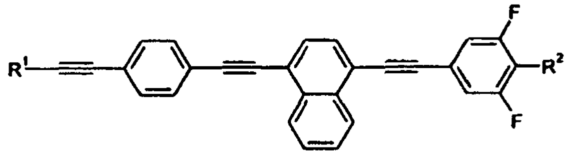

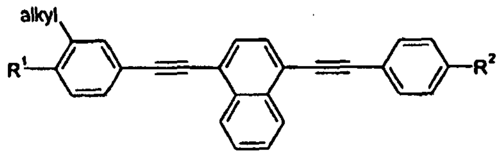

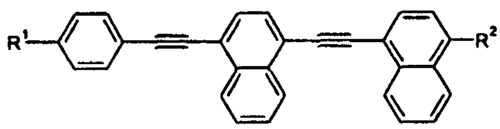

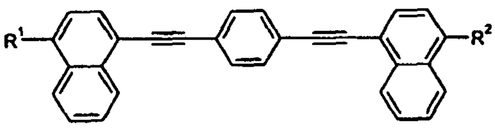

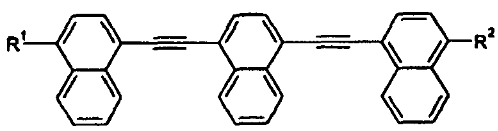

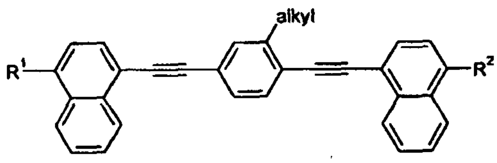

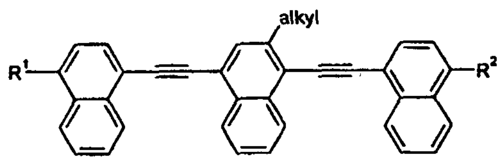

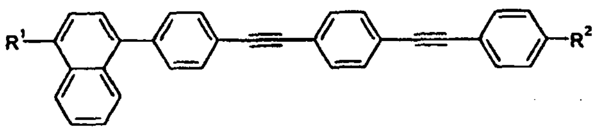

worin $R^1$ und $R^2$ wie oben definiert sind und "alkyl" eine Alkylgruppe mit 1-10 C-Atomen bedeutet.

In einer weiteren bevorzugten Ausführungsform besitzen die erfindungsgemäßen Verbindungen eine deutlich positive dielektrische Anisotropie ($\Delta\varepsilon$). Entsprechende Verbindungen haben bevorzugt eine Struktur gemäß Formel IA oder IB:

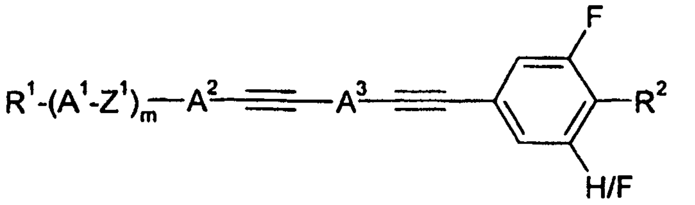

IA,

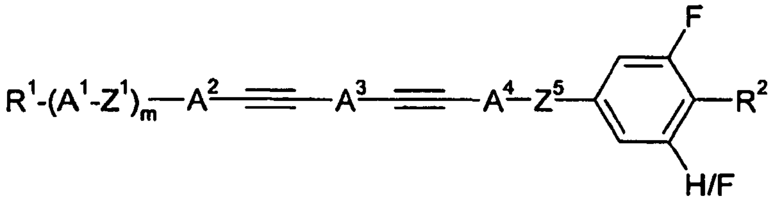

IB,

und insbesondere der Formel IA-1:

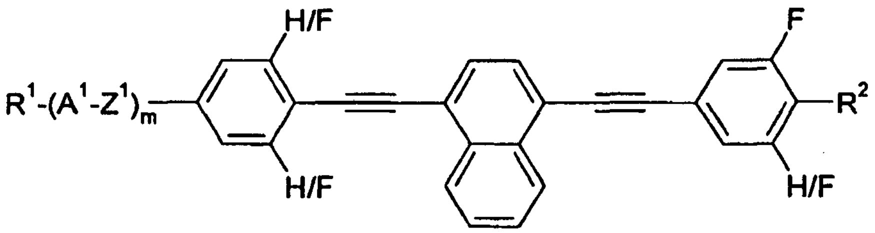

IA-1,

worin jeweils $R^1$, $A^1$, $A^2$, $A^3$, $A^4$, $Z^1$, $Z^5$ und m wie oben für Formel I definiert sind und $R^2$ F, Cl, Br, CN, $CF_3$, $OCF_3$, SCN, NCS oder $SF_5$ bedeutet.

Die Verbindungen der Formel I können vorteilhaft wie an der folgenden beispielhaften Synthese ersichtlich hergestellt werden (Schema 1):

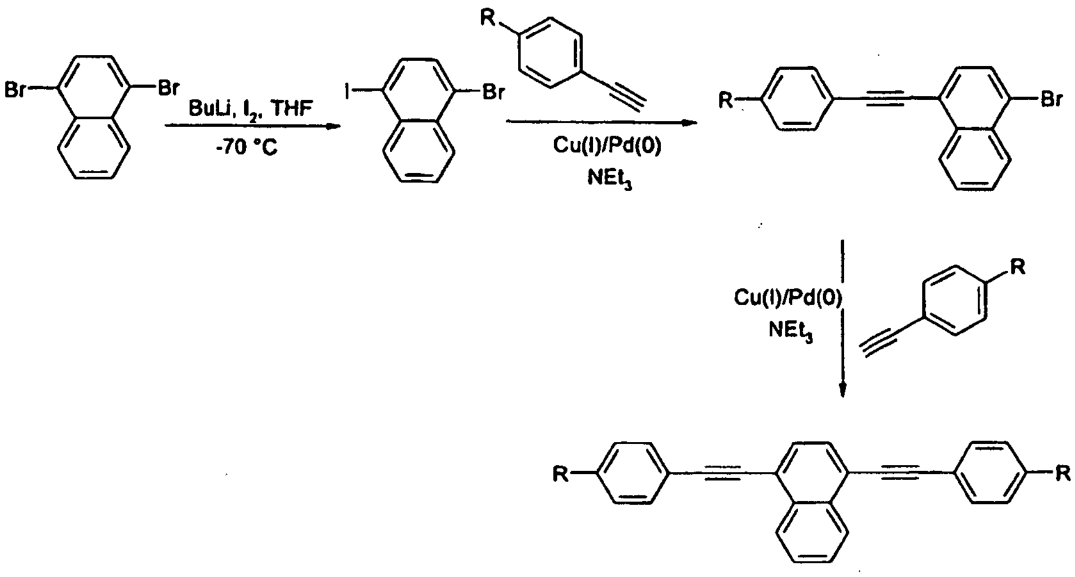


**Schema 1. Beispielhafte Synthese der Verbindungen der Formel I (asymmetrisch); R entsprechend $R^{1/2}$ definiert.**

1,4-Dibromnaphthalin wird einer Halogen-Metall-Austauschreaktion unterzogen und in 1-Iod-4-Bromnaphthalin überführt. Dieses wird in einer Sonogashira-Kupplung selektiv zunächst in die einfach funktionalisierte Acetylen-verbrückte Verbindung überführt, gefolgt von einer zweiten Sonogashira-Reaktion, wobei die Zielverbindungen der Formel

(1) mit zwei Acetylenbrücken erhalten werden. Sind die beiden Gruppen R gleich, so kann anstelle der Iodierung unmittelbar mit zwei Äquivalenten der Acetylenverbindung gekuppelt werden. Im Falle der Anthracenderivate werden entsprechende Halogenderivate als Ausgangsmaterial verwendet.

Die flüssigkristallinen Medien gemäß der vorliegenden Erfindung enthalten eine oder mehrere Verbindungen der Formel I und optional mindestens eine weitere, vorzugsweise mesogene Verbindung. Das Flüssigkristallmedium enthält daher bevorzugt zwei oder mehr Verbindungen, die vorzugsweise flüssigkristallin sind. Die Verbindungen der Formel I-X-1 und I-X-2 sind in den flüssigkristallinen Medien mit umfasst. Bevorzugte Medien umfassen die bevorzugten Verbindungen der Formel I.

Weitere Komponenten der flüssigkristallinen Medien sind vorzugsweise ausgewählt aus den Verbindungen der Formel II:

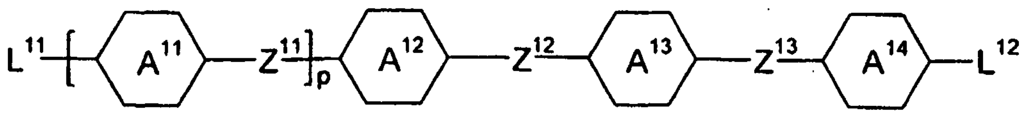
II

worin

| | |
|---|---|
| $L^{11}$ | $R^{11}$ oder $X^{11}$, |
| $L^{12}$ | $R^{12}$ oder $X^{12}$, |
| $R^{11}$ und $R^{12}$ | unabhängig voneinander unfluoriertes Alkyl oder unfluoriertes Alkoxy mit 1 bis 17, bevorzugt mit 3 bis 10, C-Atomen oder unfluoriertes Alkenyl, unfluoriertes Alkenyloxy, unfluoriertes Alkinyl oder unfluoriertes Alkoxyalkyl mit 2 bis 15, bevorzugt 3 bis 10, C-Atomen, vorzugsweise Alkyl oder unfluoriertes Alkenyl bedeuten, |
| $X^{11}$ und $X^{12}$ | unabhängig voneinander F, Cl, Br, -CN, -NCS, -SCN, -$SF_5$, fluoriertes Alkyl oder fluoriertes Alkoxy mit 1 bis 7 C-Atomen oder fluoriertes Alkenyl, fluoriertes Alkenyloxy oder fluoriertes Alkoxyalkyl mit 2 bis 7 C-Atomen, vorzugsweise fluoriertes Alkoxy, fluoriertes Alkenyloxy, F oder Cl bedeuten, und |
| p | 0 oder 1 |
| $Z^{11}$ bis $Z^{13}$ | unabhängig voneinander *trans*- -CH=CH-, *trans*--CF=CF-, -C≡C- oder eine Einfachbindung bedeuten, und |

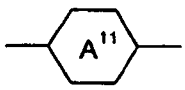
bis
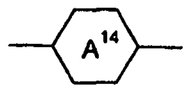

a) 1,4-Phenylen, worin ein oder mehrere, bevorzugt ein bis zwei CH-Gruppen durch N ersetzt sein können,
b) trans-1,4-Cyclohexylen oder Cyclohexenylen, worin auch eine oder zwei nicht benachbarte $CH_2$-Gruppen durch -O-und/oder -S- ersetzt sein können, und worin H durch F ersetzt sein kann,

und worin in den Gruppen a) und b)
auch ein oder mehrere H-Atome gegen Br, Cl, F, CN, -NCS, -SCN, $SF_5$, $C_1$-$C_{10}$ Alkyl, $C_1$-$C_{10}$ Alkoxy, eine ein- oder mehrfach fluorierte $C_1$-$C_{10}$ Alkyl- oder Alkoxygruppe oder eine $C_{3-6}$ Cycloalkylgruppe ersetzt sein können,

bevorzugt unabhängig voneinander

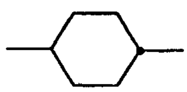
,
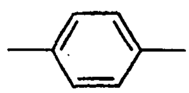
,
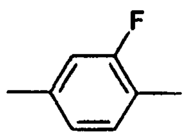
,

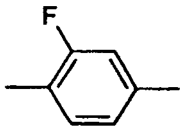, 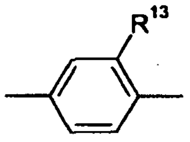, 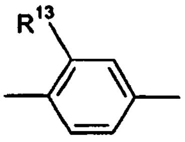,

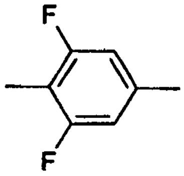, 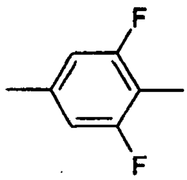 oder 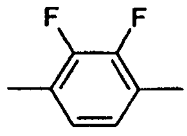,

wobei $R^{13}$ Cl, $C_{1-7}$-Alkyl oder $C_{3-6}$ Cycloalkyl bedeutet,
bedeuten.

In einer bevorzugten Ausführungsform der vorliegenden Erfindung enthalten die flüssigkristallinen Medien eine oder mehrere Verbindungen der Formel I und eine oder mehrere Verbindungen der Formel II.

Bevorzugt enthalten die flüssigkristallinen Medien gemäß der vorliegenden Anmeldung insgesamt 5 bis 95 %, bevorzugt 10 bis 90 % und besonders bevorzugt 15 bis 80 %, an Verbindungen der Formel I.

Bevorzugt enthalten die flüssigkristallinen Medien gemäß der vorliegenden Erfindung 10 % oder weniger, bevorzugt 5 % oder weniger, besonders bevorzugt 2 % oder weniger, ganz besonders bevorzugt 1 % oder weniger, und insbesondere überhaupt keine Verbindung mit nur zwei oder weniger fünf- und/oder sechsgliedrigen Ringen.

Vorzugsweise enthalten die flüssigkristallinen Medien gemäß der vorliegenden Erfindung Verbindungen ausgewählt aus der Gruppe der Verbindungen der Formeln I und II, stärker bevorzugt bestehen sie überwiegend, noch stärker bevorzugt bestehen sie im Wesentlichen und ganz bevorzugt bestehen sie vollständig daraus.

In dieser Anmeldung bedeutet "enthalten" im Zusammenhang mit Zusammensetzungen, dass die betreffende Entität, d.h. das Medium oder die Komponente, die angegebene Komponente oder Komponenten oder Verbindung oder Verbindungen enthält, vorzugsweise in einer Gesamtkonzentration von 10 % oder mehr und ganz bevorzugt von 20 % oder mehr.

"Überwiegend bestehen" bedeutet in diesem Zusammenhang, dass die betreffende Entität 55 % oder mehr, vorzugsweise 60 % oder mehr und ganz bevorzugt 70 % oder mehr der angegebenen Komponente oder Komponenten oder Verbindung oder Verbindungen enthält.

Im Wesentlichen bestehen bedeutet in diesem Zusammenhang, dass die betreffende Entität 80 % oder mehr, vorzugsweise 90 % oder mehr und ganz bevorzugt 95 % oder mehr der angegebenen Komponente oder Komponenten oder Verbindung oder Verbindungen enthält.

Vollständig bestehen bedeutet in diesem Zusammenhang, dass die betreffende Entität 98 % oder mehr, vorzugsweise 99 % oder mehr und ganz bevorzugt 100,0 % der angegebenen Komponente oder Komponenten oder Verbindung oder Verbindungen enthält.

Bevorzugt enthalten die flüssigkristallinen Medien gemäß der vorliegenden Anmeldung insgesamt 10 bis 100 %, bevorzugt 20 bis 95 % und besonders bevorzugt 25 bis 90 %, an Verbindungen der Formel I und II.

Gemäß der vorliegenden Erfindung werden die Verbindungen der Formel II vorzugsweise in einer Gesamtkonzentration von 10 % bis 90 %, stärker bevorzugt von 15 % bis 85 %, noch stärker bevorzugt von 25 % bis 80 % und ganz bevorzugt von 30 % bis 75 % der Gesamtmischung verwendet.

Die flüssigkristallinen Medien können darüber hinaus weitere Zusätze wie Stabilisatoren, chirale Dotierstoffe und Nanopartikel enthalten. Die einzelnen, zugesetzten Verbindungen werden in Konzentrationen von 0,01 bis 6 %, vorzugsweise von 0,1 bis 3 %, eingesetzt. Dabei werden jedoch die Konzentrationsangaben der übrigen Bestandteile der Flüssigkristallmischungen, also der flüssigkristallinen oder mesogenen Verbindungen, ohne Berücksichtigung der Konzentration dieser Zusatzstoffe angegeben.

Vorzugsweise enthalten die flüssigkristallinen Medien 0 bis 10 Gew.%, insbesondere 0,01 bis 5 Gew.% und besonders bevorzugt 0,1 bis 3 Gew.% an Stabilisatoren. Vorzugsweise enthalten die Medien einen oder mehrere Stabilisatoren ausgewählt aus 2,6-Di-tert-butylphenolen, 2,2,6,6-Tetramethylpiperidinen oder 2-Benzotriazol-2-yl-phenolen. Diese Hilfsstoffe sind dem Fachmann bekannt und kommerziell erhältlich, z. B. als Lichtschutzmittel.

Eine Ausführungsform der Erfindung ist daher auch ein Verfahren zur Herstellung eines Flüssigkristallmediums, das dadurch gekennzeichnet ist, dass eine oder mehrere Verbindungen der Formel I, wie in Anspruch 5 angegeben, mit einer oder mehreren Verbindungen ausgewählt aus den Verbindungen der Formel II, wie oben angegeben und optional mit einer oder mehreren weiteren Verbindungen und optional mit einem oder mehreren Additiven gemischt wird.

In der vorliegenden Anmeldung beschreibt der Ausdruck dielektrisch positiv Verbindungen oder Komponenten mit $\Delta\varepsilon > 3{,}0$, dielektrisch neutral mit $-1{,}5 \leq \Delta\varepsilon \leq 3{,}0$ und dielektrisch negativ mit $\Delta\varepsilon < -1{,}5$. $\Delta\varepsilon$ wird bei einer Frequenz von

1 kHz und 20°C bestimmt. Die dielektrische Anisotropie der jeweiligen Verbindung wird aus den Ergebnissen einer Lösung von 10 % der jeweiligen einzelnen Verbindung in einer nematischen Host-Mischung bestimmt. Wenn die Löslichkeit der jeweiligen Verbindung in der Host-Mischung weniger als 10 % beträgt, wird die Konzentration auf 5 % reduziert. Die Kapazitäten der Testmischungen werden sowohl in einer Zelle mit homeotroper als auch mit homogener Orientierung bestimmt. Die Schichtdicke beträgt bei beiden Zelltypen ca. 20 μm. Die angelegte Spannung ist eine Rechteckwelle mit einer Frequenz von 1 kHz und einem Effektivwert von typischerweise 0,5 V bis 1,0 V, wird jedoch stets so ausgewählt, dass sie unterhalb der kapazitiven Schwelle für die jeweilige Testmischung liegt.

$\Delta\varepsilon$ ist als ($\varepsilon_{\parallel}$-$\varepsilon\perp$) definiert, während $\varepsilon_{Drchschn}$ ($\varepsilon_{\parallel}$ + 2 $\varepsilon_{\perp}$) /3 ist.

Als Host-Mischung wird für dielektrisch positive Verbindungen die Mischung ZLI-4792 und für dielektrisch neutrale sowie für dielektrisch negative Verbindungen die Mischung ZLI-3086 verwendet, beide von Merck KGaA, Deutschland. Die absoluten Werte der dielektrischen Konstanten der Verbindungen werden aus der Änderung der jeweiligen Werte der Host-Mischung bei Zugabe der interessierenden Verbindungen bestimmt. Die Werte werden auf eine Konzentration der interessierenden Verbindungen von 100 % extrapoliert.

Komponenten, die bei der Messtemperatur von 20°C eine nematische Phase aufweisen, werden als solche gemessen, alle anderen werden wie Verbindungen behandelt.

Der Ausdruck Schwellenspannung bezeichnet in der vorliegenden Anmeldung die optische Schwelle und ist für 10 % relativen Kontrast ($V_{10}$) angegeben, der Ausdruck Sättigungsspannung bezeichnet die optische Sättigung und ist für 90 % relativen Kontrast ($V_{90}$) angegeben, in beiden Fällen, soweit nicht ausdrücklich etwas anderes angegeben ist. Die kapazitive Schwellenspannung ($V_0$), auch Freedericks-Schwelle $V_{Fr}$ genannt, wird nur verwendet, wenn dies ausdrücklich genannt ist.

Die in dieser Anmeldung angegebenen Parameterbereiche schließen sämtlich die Grenzwerte ein, wenn nicht ausdrücklich etwas anderes angegeben ist.

Die unterschiedlichen für verschiedene Bereiche von Eigenschaften angegebenen oberen und unteren Grenzwerte ergeben in Kombination miteinander zusätzliche bevorzugte Bereiche.

In der gesamten Anmeldung gelten, wenn nicht ausdrücklich anders angegeben, die folgenden Bedingungen und Definitionen. Alle Konzentrationen sind in Massenprozent angegeben und beziehen sich jeweils auf die Gesamtmischung, alle Temperaturen und alle Temperaturunterschiede sind in Grad Celsius bzw. Differenzgrad angegeben. Alle für Flüssigkristalle typischen physikalischen Eigenschaften werden nach "Merck Liquid Crystals, Physical Properties of Liquid Crystals", Stand Nov. 1997, Merck KGaA, Deutschland, bestimmt und sind für eine Temperatur von 20°C aufgeführt, wenn nicht ausdrücklich anders angegeben. Die optische Anisotropie ($\Delta n$) wird bei einer Wellenlänge von 589,3 nm bestimmt. Die dielektrische Anisotropie ($\Delta\varepsilon$) wird bei einer Frequenz von 1 kHz bestimmt. Die Schwellenspannungen sowie alle anderen elektrooptischen Eigenschaften werden mit bei Merck KGaA, Deutschland, hergestellten Testzellen bestimmt. Die Testzellen für die Bestimmung von $\Delta\varepsilon$ besitzen eine Schichtdicke von circa 20 μm. Bei der Elektrode handelt es sich um eine kreisförmige ITO-Elektrode mit einer Fläche von 1,13 $cm^2$ und einem Schutzring. Die Ausrichtungsschichten sind SE-1211 von Nissan Chemicals, Japan, für homeotrope Ausrichtung ($\varepsilon_{\parallel}$) und Polyimid AL-1054 von Japan Synthetic Rubber, Japan, für homogene Ausrichtung ($\varepsilon_{\perp}$). Die Bestimmung der Kapazitäten erfolgt mit einem Frequenzgang-Analysegerät Solatron 1260 unter Verwendung einer Sinuswelle mit einer Spannung von 0,3 $V_{rms}$. Als Licht wird bei den elektrooptischen Messungen weißes Licht verwendet. Dabei wird ein Aufbau mit einem im Handel erhältlichen Gerät DMS der Fa. Autronic-Melchers, Germany verwendet. Die charakteristischen Spannungen werden unter senkrechter Beobachtung bestimmt. Die Schwellenspannung ($V_{10}$), "Mittgrau-Spannung" ($V_{50}$) und Sättigungsspannung ($V_{90}$) werden für 10 %, 50 % bzw. 90 % relativen Kontrast bestimmt.

Die flüssigkristallinen Medien werden bezüglich ihrer Eigenschaften im Frequenzbereich der Mikrowellen untersucht wie in A. Penirschke, S. Müller, P. Scheele, C. Weil, M. Wittek, C. Hock und R. Jakoby: "Cavity Perturbation Method for Characterization of Liquid Crystals up to 35GHz", 34th European Microwave Conference - Amsterdam, S. 545-548 beschreiben.

Vergleiche hierzu auch A. Gaebler, F. Gölden, S. Müller, A. Penirschke und R. Jakoby "Direct Simulation of Material Permittivites ...", 12MTC 2009 - International Instrumentation and Measurement Technology Conference, Singapur, 2009 (IEEE), S. 463-467 und DE 10 2004 029 429 A, in der ebenfalls detailliert ein Meßverfahren beschrieben wird.

Der Flüssigkristall wird beispielsweise in eine Kapillare aus Polytetrafuorethylen (PTFE) oder Quarzglas gefüllt. Die Kapillare hat einen inneren Radius von 180 μm und einen äußeren Radius von 350 μm. Die effektive Länge beträgt 2,0 cm. Die gefüllte Kapillare wird in die Mitte der Kavität mit einer Resonanzfrequenz von 30 GHz eingebracht. Diese Kavität hat eine Länge von 6,6 mm, eine Breite von 7,1 mm und eine Höhe von 3,6 mm. Daraufhin wird das Eingangssignal ("source") angelegt und das Ergebnis des Ausgangssignals mit einem kommerziellen Netzwerkanalysator ("vector network analyzer") aufgenommen. Für andere Frequenzen (z.B. 19 GHz) werden die Abmessungen der Kavität entsprechend angepasst.

Aus der Änderung der Resonanzfrequenz und des Q-Faktors, zwischen der Messung mit der mit dem Flüssigkristall gefüllten Kapillare und der Messung ohne der mit dem Flüssigkristall gefüllten Kapillare, wird die dielektrische Konstante und der Verlustwinkel bei der entsprechenden Zielfrequenz mittels der Gleichungen 10 und 11 der A. Penirschke, S. Müller, P. Scheele, C. Weil, M. Wittek, C. Hock und R. Jakoby: "Cavity Perturbation Method for Characterization of Liquid Crystals up to 35 GHz", 34th European Microwave Conference - Amsterdam, S. 545-548 bestimmt, wie dort beschrieben.

Die Werte für die Komponenten der Eigenschaften senkrecht bzw. and parallel zum Direktor des Flüssigkristalls werden durch Orientierung des Flüssigkristalls in einem Magnetfeld erhalten. Dazu wird das Magnetfeld eines Permanentmagneten verwendet. Die Stärke des Magnetfelds beträgt 0,35 Tesla. Die Orientierung des Magneten wird entsprechend eingestellt und dann entsprechend um 90° gedreht.

Die dielektrische Anisotropie im $\mu$-Wellenbereich ist definiert als

$$\Delta\varepsilon_r \equiv (\varepsilon_{r,||} - \varepsilon_{r,\perp}) .$$

Die Modulierbarkeit bzw. Steuerbarkeit ("tuneability", $\tau$) ist definiert als

$$\tau \equiv (\Delta\varepsilon_r / \varepsilon_{r,||}) .$$

Die Materialgüte ($\eta$) ist definiert als

$$\eta \equiv (\tau / \tan \delta_{\varepsilon\, r,Max.}) ,$$

mit dem maximalen dielektrischen Verlustfaktor tan $\delta_{\varepsilon\, r,Max}$:

$$\tan \delta_{\varepsilon\, r,Max.} \equiv \mathrm{Max.}\ \{ \tan \delta_{\varepsilon\, r,\perp};\ \tan \delta_{\varepsilon\, r,||} \}$$

der aus dem Maximalwert der gemessenen Werte für tan$\delta_\varepsilon$, hervorgeht.

Die Materialgüte ($\eta$) der bevorzugten Flüssigkristallmaterialien beträgt 5 oder mehr, bevorzugt 6 oder mehr, bevorzugt 8 oder mehr, bevorzugt 10 oder mehr, bevorzugt 15 oder mehr, bevorzugt 17 oder mehr, besonders bevorzugt 20 oder mehr und ganz besonders bevorzugt 25 oder mehr.

Die bevorzugten Flüssigkristallmaterialien haben in den entsprechenden Bauteilen Phasenschiebergüten von 15°/dB oder mehr, bevorzugt von 20°/dB oder mehr, bevorzugt von 30°/dB oder mehr, bevorzugt von 40°/dB oder mehr, bevorzugt von 50°/dB oder mehr, besonders bevorzugt von 80°/dB oder mehr und ganz besonders bevorzugt von 100°/dB oder mehr.

In der vorliegenden Anmeldung, bedeutet, wenn nicht ausdrücklich anders angegeben, der Begriff Verbindungen sowohl eine Verbindung, als auch mehrere Verbindungen.

Die erfindungsgemäßen Flüssigkristallmedien weisen bevorzugt nematische Phasen von jeweils mindestens von -20°C bis 80°C, bevorzugt von -30°C bis 85°C und ganz besonders bevorzugt von -40°C bis 100°C auf. Insbesondere bevorzugt reicht die Phase bis 120°C oder mehr, bevorzugt bis 140°C oder mehr und ganz besonders bevorzugt bis 180°C oder mehr. Hierbei bedeutet der Begriff eine nematische Phase aufweisen einerseits, dass bei tiefen Temperaturen bei der entsprechenden Temperatur keine smektische Phase und keine Kristallisation beobachtet wird und andererseits, dass beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen, mit einer Schichtdicke von 5 $\mu$m, für mindestens 100 Stunden überprüft. Bei hohen Temperaturen wird der Klärpunkt nach üblichen Methoden in Kapillaren gemessen.

Die Flüssigkristallmedien gemäß der vorliegenden Erfindung weisen bevorzugt einen Klärpunkt von 90°C oder mehr, stärker bevorzugt von 100°C oder mehr, noch stärker bevorzugt von 120°C oder mehr, besonders bevorzugt von 150°C oder mehr und ganz besonders bevorzugt von 170°C oder mehr, auf.

Das $\Delta\varepsilon$ des Flüssigkristallmediums gemäß der Erfindung bei 1 kHz und 20°C beträgt vorzugsweise 1 oder mehr, stärker bevorzugt 2 oder mehr und ganz bevorzugt 3 oder mehr.

Das $\Delta$n der Flüssigkristallmedien gemäß der vorliegenden Erfindung liegt bei 589 nm ($Na^D$) und 20°C vorzugsweise im Bereich von 0,20 oder mehr bis 0,90 oder weniger, stärker bevorzugt im Bereich von 0,25 oder mehr bis 0,90 oder weniger, noch stärker bevorzugt im Bereich von 0,30 oder mehr bis 0, 85 oder weniger und ganz besonders bevorzugt im Bereich von 0,35 oder mehr bis 0,80 oder weniger.

In einer bevorzugten Ausführungsform der vorliegenden Anmeldung beträgt das $\Delta$n der Flüssigkristallmedien gemäß der vorliegenden Erfindung vorzugsweise 0,50 oder mehr, stärker bevorzugt 0,55 oder mehr.

Ferner sind die erfindungsgemäßen Flüssigkristallmedien durch hohe Anisotropien im Miktowellenbereich gekennzeichnet. Die Doppelbrechung beträgt z.B. bei ca. 8,3 GHz bevorzugt 0,14 oder mehr, besonders bevorzugt 0,15 oder mehr, besonders bevorzugt 0,20 oder mehr, besonders bevorzugt 0,25 oder mehr und ganz besonders bevorzugt 0,30 oder mehr. Außerdem beträgt die Doppelbrechung bevorzugt 0,80 oder weniger.

In einigen Ausführungsformen können jedoch auch Flüssigkristalle mit einem negativen Wert der dielektrischen Anisotropie vorteilhaft verwendet werden.

Die eingesetzten Flüssigkristalle sind entweder Einzelsubstanzen oder Mischungen. Bevorzugt weisen sie eine nematische Phase auf.

Bevorzugte Bauelemente, die ein Flüssigkristallmedium oder wenigstens eine Verbindung gemäß der Erfindung enthalten, sind Phasenschieber, Varaktoren, Antennenarrays (z. B. für Funk, Mobilfunk, Radio, Mikrowellen/Radar und sonstige Datenübertragung), 'matching circuit adaptive filters' und andere. Bevorzugt sind Bauteile für die Hochfrequenztechnik, wie oben definiert. Bevorzugt sind außerdem durch unterschiedliche angelegte elektrische Spannungen modulierbare Bauteile. Ganz besonders bevorzugte Bauelemente sind Phasenschieber. In bevorzugten Ausführungsformen werden mehrere Phasenschieber funktionell verbunden, wodurch beispielsweise ein phasengesteuerte Gruppenantenne resultiert. Eine Gruppenantenne nutzt die Phasenverschiebung der in einer Matrix angeordneten Sende- oder Empfangselemente, um durch Interferenz eine Bündelung zu erzielen. Aus einer reihen- oder gitterförmigen parallelen Anordnung von Phasenschiebern lässt sich ein sog. 'phased array' aufbauen, das als abstimmbare Sende- oder Empfangsantenne für Hochfrequenzen (z. B. Gigahertzbereich) dienen kann. Erfindungsgemäße 'phased array'-Antennen besitzen einen sehr breiten nutzbaren Empfangskegel.

Bevorzugte Anwendungen sind Radarinstallationen und Datenübertragungsgeräte auf bemannten oder unbemannten Fahrzeugen aus dem Bereich Automobil, Schifffahrt, Flugzeuge, Raumfahrt und Satellitentechnik.

Zur Herstellung geeigneter Bauelemente, insbesondere Phasenschieber, wird typischerweise ein erfindungsgemäßes flüssigkristallines Medium in rechteckige Kavitäten von weniger als 1 mm Querschnitt und mehreren Zentimetern Länge eingebracht. Die Kavitäten besitzen entlang zweier langer Seiten angebrachte, gegenüberliegende Elektroden. Solche Anordnungen sind dem Fachmann vertraut. Durch Anlegen einer variablen Spannung können im späteren Betrieb die dielektrischen Eigenschaften des flüssigkristallinen Mediums abgestimmt werden, um verschiedene Frequenzen oder Richtungen einer Antenne einzustellen.

Der Ausdruck "Alkyl" umfasst vorzugsweise geradkettige und verzweigte Alkylgruppen mit 1 bis 15 Kohlenstoffatomen, insbesondere die geradkettigen Gruppen Methyl, Ethyl, Propyl, Butyl, Pentyl, Hexyl und Heptyl. Gruppen mit 2 bis 10 Kohlenstoffatomen sind im Allgemeinen bevorzugt.

Der Ausdruck "Alkenyl" umfasst vorzugsweise geradkettige und verzweigte Alkenylgruppen mit 2 bis 15 Kohlenstoffatomen, insbesondere die geradkettigen Gruppen. Besonders bevorzugte Alkenylgruppen sind $C_2$ bis $C_7$-1E-Alkenyl, $C_4$ bis $C_7$-3E-Alkenyl, $C_5$ bis $C_7$-4-Alkenyl, $C_6$ bis $C_7$-5-Alkenyl und $C_7$-6-Alkenyl, insbesondere $C_2$ bis $C_7$-1E-Alkenyl, $C_4$ bis $C_7$-3E-Alkenyl und $C_5$ bis $C_7$-4-Alkenyl. Beispiele weiterer bevorzugter Alkenylgruppen sind Vinyl, 1 E-Propenyl, 1 E-Butenyl, 1E-Pentenyl, 1 E-Hexenyl, 1 E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Alkoxy" umfasst vorzugsweise geradkettige Reste der Formel $C_nH_{2n+1}$-O-, worin n 1 bis 10 bedeuten. Vorzugsweise ist n 1 bis 6. Bevorzugte Alkoxygruppen sind beispielsweise Methoxy, Ethoxy, n-Propoxy, n-Butoxy, n-Pentoxy, n-Hexoxy, n-Heptoxy, n-Octoxy, n-Nonoxy, n-Decoxy.

Der Ausdruck "Oxaalkyl" bzw. "Alkoxyalkyl" umfasst vorzugsweise geradkettige Reste der Formel $C_nH_{2n+1}$-O-$(CH_2)_m$, worin n und m jeweils unabhängig voneinander 1 bis 10 bedeuten. Vorzugsweise ist n 1 und m 1 bis 6.

Der Ausdruck "fluorierter Alkylrest" umfasst vorzugsweise ein- oder mehrfach fluorierte Reste. Perfluorierte Reste sind eingeschlossen. Besonders bevorzugt sind $CF_3$, $CH_2CF_3$, $CH_2CHF_2$, $CHF_2$, $CH_2F$, $CHFCF_3$ und $CF_2CHFCF_3$.

In der vorliegenden Anmeldung bedeutet Hochfrequenztechnik Anwendungen mit Frequenzen im Bereich von 1 MHz bis 1 THz, bevorzugt von 1 GHz bis 500GHz, stärker bevorzugt 2 GHz bis 300 GHz, insbesondere bevorzugt von 5 bis 150 GHz.

Die Flüssigkristallmedien gemäß der vorliegenden Erfindung können weitere Zusatzstoffe und chirale Dotierstoffe in den üblichen Konzentrationen beinhalten. Die Gesamtkonzentration dieser weiteren Bestandteile liegt im Bereich von 0 % bis 10 %, vorzugsweise 0,1 % bis 6 %, bezogen auf die Gesamtmischung. Die Konzentrationen der einzelnen verwendeten Verbindungen liegen vorzugsweise jeweils im Bereich von 0,1 % bis 3 %. Die Konzentration dieser und ähnlicher Zusatzstoffe wird bei der Angabe der Werte und Konzentrationsbereiche der Flüssigkristallkomponenten und Flüssigkristallverbindungen der Flüssigkristallmedien in dieser Anmeldung nicht berücksichtigt.

Die erfindungsgemäßen Flüssigkristallmedien bestehen aus mehreren Verbindungen, vorzugsweise aus 3 bis 30, stärker bevorzugt aus 4 bis 20 und ganz bevorzugt aus 4 bis 16 Verbindungen. Diese Verbindungen werden auf herkömmliche Weise gemischt. In der Regel wird die gewünschte Menge der in der geringeren Menge verwendeten Verbindung in der in der größeren Menge verwendeten Verbindung gelöst. Liegt die Temperatur über dem Klärpunkt der in der höheren Konzentration verwendeten Verbindung, ist die Vervollständigung des Lösungsvorgangs besonders

leicht zu beobachten. Es ist jedoch auch möglich, die Medien auf anderen üblichen Wegen, beispielsweise unter Verwendung von so genannten Vormischungen, bei denen es sich z.B. um homologe oder eutektische Mischungen von Verbindungen handeln kann, oder unter Verwendung von so genannten "Multi-Bottle"-Systemen, deren Bestandteile selbst gebrauchsfertige Mischungen sind, herzustellen.
Alle Temperaturen, wie z.B. der Schmelzpunkt T(K,N) bzw. T(K,S), der Übergang von der smektischen (S) zur nematischen (N) Phase T(S,N) und der Klärpunkt T (N,I) der Flüssigkristalle sind in Grad Celsius angegeben. Alle Temperaturdifferenzen sind in Differenzgraden angegeben.

In der vorliegenden Anmeldung bedeutet Hochfrequenztechnik Anwendungen mit Frequenzen im Bereich von 1 MHz bis 1 THz, bevorzugt von 1 GHz bis 500 GHz, bevorzugt 2 GHz bis 300 GHz, insbesondere bevorzugt von ca. 5 bis 150 GHz. Die Anwendung liegt bevorzugt im Mikrowellenspektrum oder angrenzenden, für die Nachrichtenübertragung geeigneten Bereichen, in denen 'phased array'-Module in Sende- und Empfangsantennen zum Einsatz kommen können.

In der vorliegenden Anmeldung und in den folgenden Beispielen sind die Strukturen der Flüssigkristallverbindungen durch Akronyme angegeben, wobei die Transformation in chemische Formeln gemäß folgender Tabellen A und B erfolgt. Alle Reste $C_nH_{2n+1}$ und $C_mH_{2m+1}$ sind geradkettige Alkylreste mit n bzw. m C-Atomen; n, m und k sind ganze Zahlen und bedeuten vorzugsweise 0, 1, 2, 3, 4, 5, 6, 7, 8, 9, 10, 11 oder 12. Die Codierung gemäß Tabelle B versteht sich von selbst. In Tabelle A ist nur das Akronym für den Grundkörper angegeben. Im Einzelfall folgt getrennt von Akronym für den Grundkörper mit einem Strich ein Code für die Substituenten $R^{1*}$, $R^{2*}$, $L^{1*}$ und $L^{2*}$:

| Code für $R^{1*}$, $R^{2*}$, $L^{1*}$, $L^{2*}$, $L^{3*}$ | $R^{1*}$ | $R^{2*}$ | $L^{1*}$ | $L^{2*}$ |
|---|---|---|---|---|
| nm | $C_nH_{2n+1}$ | $C_mH_{2m+1}$ | H | H |
| nOm | $C_nH_{2n+1}$ | $OC_mH_{2m+1}$ | H | H |
| nO.m | $OC_nH_{2n+1}$ | $C_mH_{2m+1}$ | H | H |
| n | $C_nH_{2n+1}$ | CN | H | H |
| nN.F | $C_nH_{2n+1}$ | CN | F | H |
| nN.F.F | $C_nH_{2n+1}$ | CN | F | F |
| nF | $C_nH_{2n+1}$ | F | H | H |
| nCl | $C_nH_{2n+1}$ | Cl | H | H |
| nOF | $OC_nH_{2n+1}$ | F | H | H |
| nF.F | $C_nH_{2n+1}$ | F | F | H |
| nF.F.F | $C_nH_{2n+1}$ | F | F | F |
| $nOCF_3$ | $C_nH_{2n+1}$ | $OCF_3$ | H | H |
| $nOCF_3$.F | $C_nH_{2n+1}$ | $OCF_3$ | F | H |
| n-Vm | $C_nH_{2n+1}$ | $-CH=CH-C_mH_{2m+1}$ | H | H |
| nV-Vm | $C_nH_{2n+1}-CH=CH-$ | $-CH=CH-C_mH_{2m+1}$ | H | H |

Geeignete Mischungskomponenten finden sich in den Tabellen A und B.

Tabelle A

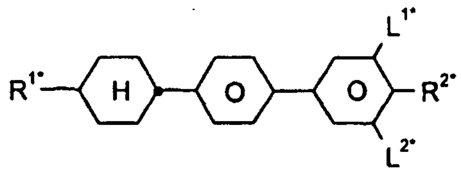

BCH

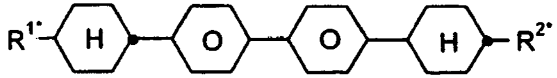

CBC

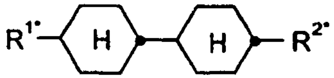

CCH

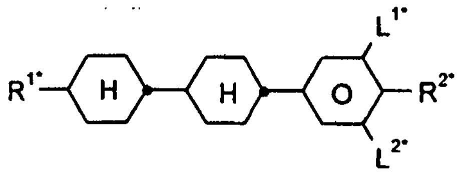

CCP

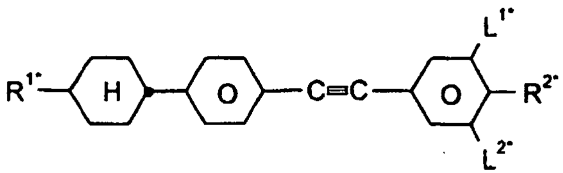

CPTP

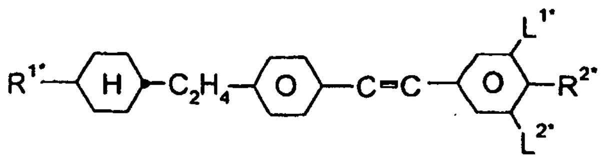

CEPTP

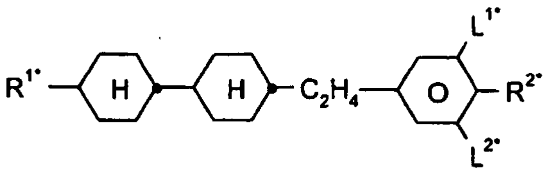

ECCP

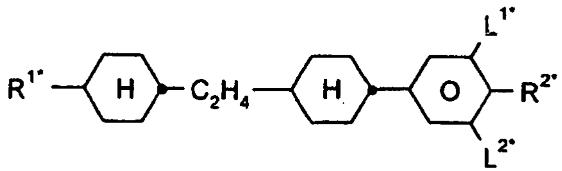

CECP

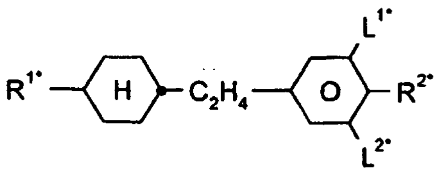

EPCH

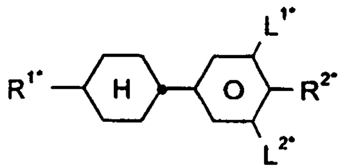

PCH

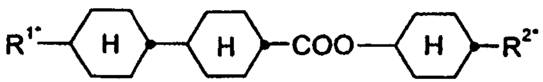

(fortgesetzt)

CH

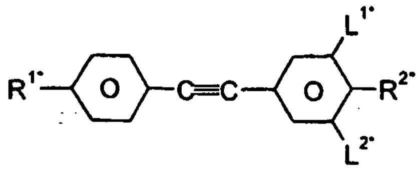

PTP

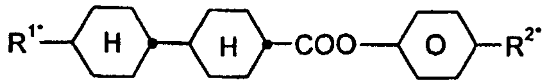

CP

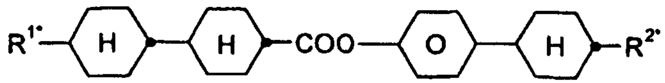

CCPC

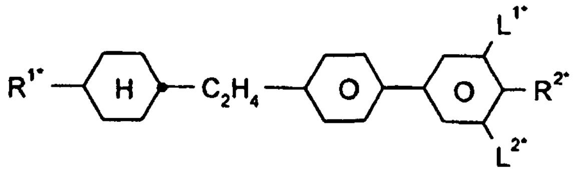

BECH

Tabelle B

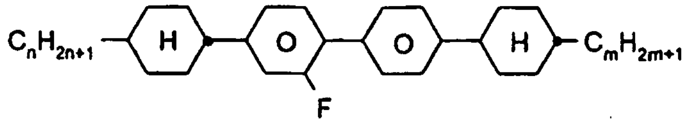

CBC-nmF

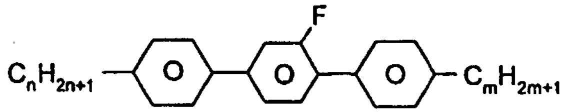

PGP-n-m

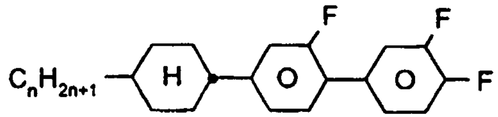

CGG-n-F

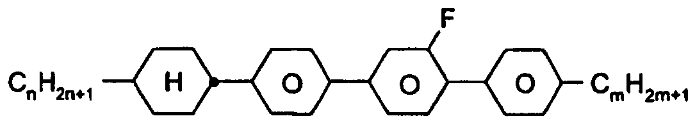

CPGP-n-m

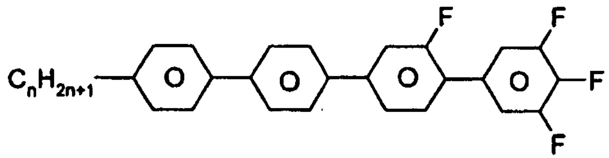

PPGU-n-F

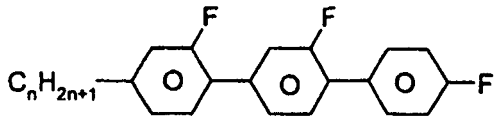

GGP-n-F

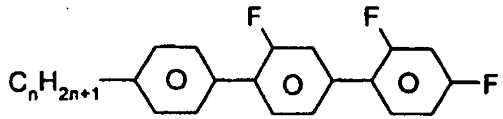

PGIGI-n-F

Die folgenden Beispiele erläutern die vorliegende Erfindung, ohne sie in irgendeiner Weise zu beschränken.

Aus den physikalischen Eigenschaften wird dem Fachmann jedoch deutlich, welche Eigenschaften zu erzielen sind und in welchen Bereichen sie modifizierbar sind. Insbesondere ist also die Kombination der verschiedenen Eigenschaften, die vorzugsweise erreicht werden können, für den Fachmann gut definiert.

**Beispiele**

Die eingesetzten Acetylene, soweit nicht kommerziell erhältlich, werden nach Standard-Laborvorschriften synthetisiert.

Synthesebeispiele:

1) Synthese von 1-Iod-4-Bromnaphthalin

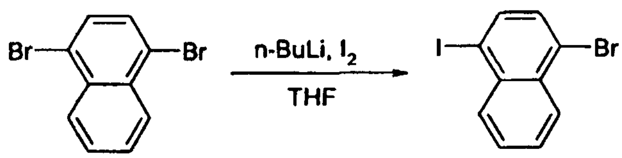


100 g (350 mmol) 1,4-Dibromnaphthalin werden in 1 l THF vorgelegt, auf -70 °C gekühlt und tropfenweise mit 235 ml n-BuLi (1,6 M in Hexan, 370 mmol) versetzt. Nach 1 h werden 103 g $I_2$ (406 mmol) in 250 ml THF zugetropft, 2 h bei -70 °C nachgerührt, auf 0 °C erwärmt und durch die Zugabe von 50 ml (644 mmol) wässriger $NaHSO_3$-Lösung (w = 39 %) gequencht. Die Phasen werden getrennt und die wässrige Phase einmal mit MTB extrahiert. Die vereinigten organischen Phasen werden mit gesättigter Natriumchloridlsg. gewaschen, über Natriumsulfat getrocknet, abfiltriert und am Rotationsverdampfer eingeengt. Der Rückstand wird säulenchromatographisch ($SiO_2$, Heptan) gereinigt, die weitere Aufreinigung erfolgt durch Umkristallisation aus Isopropanol. 1-Iod-4-Bromnaphthalin wird als gelber Feststoff erhalten.

2) Synthese von 1-Brom-4-(4-n-propyl-phenylethynyl)-naphthalin

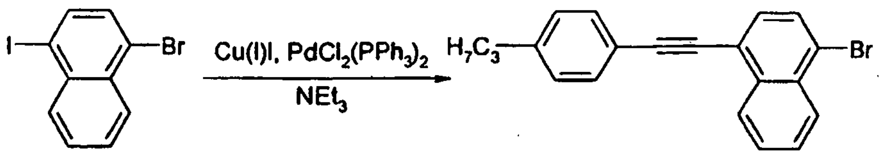


15,3 g (43,6 mmol) 1-Iod-4-Bromnaphthalin und 7,25 g (5,3 mmol) 4-n-Propylphenylacetylen werden in 200 ml $NEt_3$ vorgelegt, mit 170 mg (0,9 mmol) Kupfer(I)iodid und 600 mg (0,9 mmol) Bis(triphenylphosphin)-palladium(II)-chlorid versetzt und 30 Minuten refluxiert. Der Ansatz wird abgekühlt, mit Wasser und Heptan versetzt und die Phasen getrennt. Die organische Phase wird mit gesättigter Natriumchloridlsg. gewaschen, über Natriumsulfat getrocknet, abfiltriert und am Rotationsverdampfer eingeengt. Der Rückstand wird säulenchromatographisch ($SiO_2$, Heptan) gereinigt, die weitere Aufreinigung erfolgt durch Umkristallisation aus Isopropanol.

3) Synthese von 1-(4-n-Butyl-phenylethynyl)-4-(4-n-propyl-phenylethynyl)-naphthalin

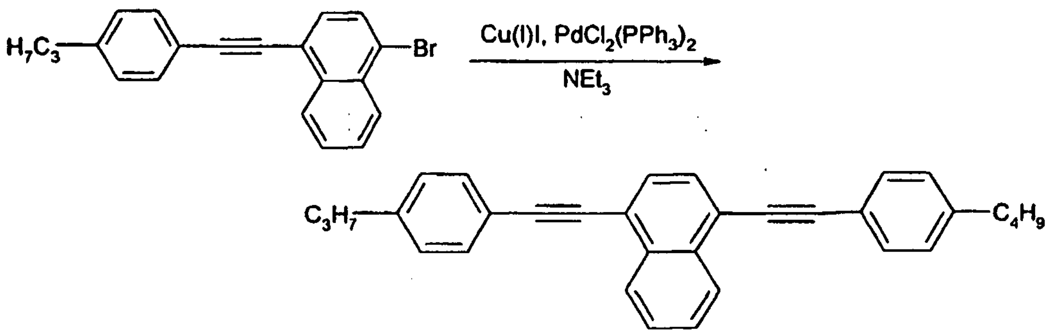


2,35 g (6,3 mmol) 1-Brom-4-(4-n-propyl-phenylethynyl)-naphthalin und 1,33 g (8,4 mmol) 4-n-Butylphenylacetylen werden in 40 ml $NEt_3$ vorgelegt, mit 60 mg (0,3 mmol) Kupfer(I)iodid und 200 mg (0,3 mmol) Bis(triphenylphosphin)-palladium(II)-chlorid versetzt und 18 h refluxiert. Der Ansatz wird abgekühlt, mit Wasser und Heptan versetzt und die Phasen getrennt. Die organische Phase wird mit gesättigter Ammoniumchloridlösung und anschließend mit gesättigter Natriumchloridlsg. gewaschen, über Natriumsulfat getrocknet, abfiltriert und am Rotationsverdampfer eingeengt. Der Rückstand der Verbindung (1) wird säulenchromatographisch ($SiO_2$, Heptan) gereinigt, die weitere Aufreinigung erfolgt durch Umkristallisation aus Isopropanol.

**MS (EI)**: m/z(%) = 426 (100, $M^+$), 397 (11, $[M - Ethy]^+$), 383 (16, $[M - Propyl]^+$), 354 (18, $[M - Ethy-Propyl]^+$), 177 (14, $[M - Ethy-Propyl]^{2+}$).

$\Delta\varepsilon$ = +1,7
$\Delta n$ = 0,42
$\gamma_1$ = 1283 mPa·s
K 78 N 191 I

Analog werden synthetisiert:

4) 1,4-Bis-(4-n-butyl-phenylethynyl)-naphthalin (1)

Analog zu Beispiel 3 wird die Titelverbindung aus 1,4-Dibromnaphthalin und zwei Äquivalenten 4-n-Butylphenylacetylen hergestellt.

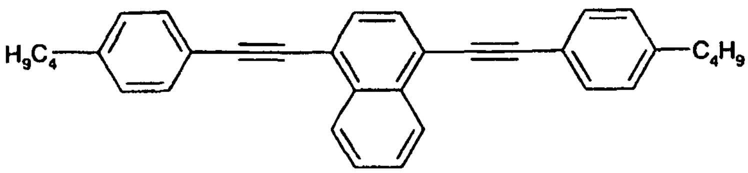

**(1)**

**MS (EI)**: m/z(%) = 440 (100, $M^+$), 397 (31, $[M - Propyl]^+$), 354 (21, $[M - Propyl-Propyl]^+$), 177 (9, $[M - Propyl-Propyl]^{2+}$).

$\Delta\varepsilon$ = +1,2
$\Delta n$ = 0,41
$\gamma_1$ = 1433 mPa·s
K 75 N 176 I

5) 1-(4-n-Hexyl-phenylethynyl)-4-(4-n-propyl-phenylethynyl)-naphthalin

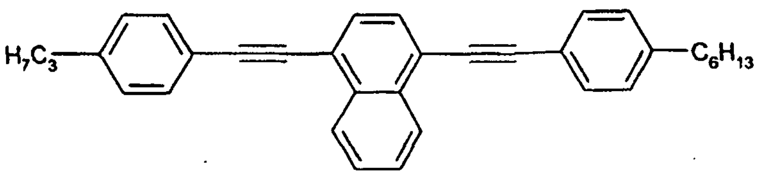

**MS (EI)**: m/z(%) = 454 (100, $M^+$), 425 (8, $[M - Ethyl]^+$), 383 (22, $[M - Pentyl]^+$), 354 (20, $[M - Pentyl\text{-}Ethyl]^+$), 177 (7, $[M - Pentyl\text{-}Ethyl]^{2+}$).

$\Delta\varepsilon$ =+1,2
$\Delta n$ = 0,41
$\gamma_1$ = 2067 mPa·s
K 63 N 172 I

6) 1-(4-Fluor-phenylethynyl)-4-(4-n-propyl-phenylethynyl)-naphthalin

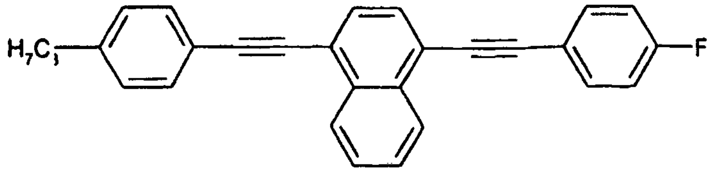

**MS (EI)**: m/z(%) = 388 (100, $M^+$), 359 (55, $[M - Ethyl]^+$), 179,5 (14, $[M - Ethyl]^{2+}$).

$\Delta\varepsilon$ = +5,2

$\Delta n$ = 0,43

$\gamma_1$ = 1782 mPa·s

K 103 N 188 I

7) 1-(5-Butyl-thiophen-2-ethynyl)-4-(4-n-propyl phenylethynyl)-naphthalin

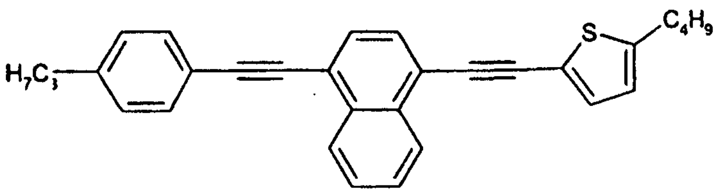

**MS (EI)**: m/z(%) = 432 (100, $M^+$), 389 (44, $[M - Propyl]^+$), 360 (14, $[M - Propyl\text{-}Ethyl]^+$), 180 (14, $[M -Propyl\text{-}Ethyl]^{2+}$).

$\Delta\varepsilon$ = +2,2

$\Delta n$ = 0,44

$\gamma 1$ = 1353 mPa·s

K 67 N 107 I

8) 1,4-Bis-(4-n-Butyl-Phenylethynyl)-2-methylnaphthalin

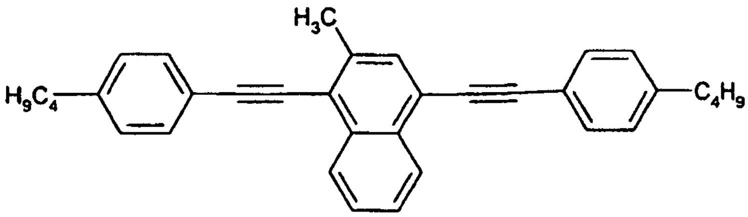

**MS (EI)**: m/z(%) = 454 (100, $M^+$), 411 (21, $[M - Propyl]^+$), 368 (8 $[M - Propyl-Propyl]^+$), 184 $[M - Propyl-Propyl]^{2+}$).

$\Delta\varepsilon = +1{,}2$

$\Delta n = 0{,}40$

$\gamma_1$ = 3157 mPa·s

K 95 N 1338 I

9) 1-(2,3-Difluor-4-ethoxy-phenylethynyl)-4-(4-n-butyl-phenylethnyl)-naphthalin

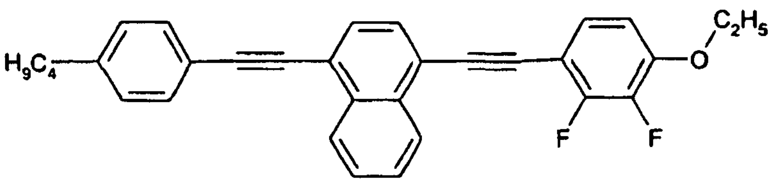

**MS (EI)**: m/z(%) = 464 (100, $M^+$), 435 (12, $[M - Ethyl]^+$), 421 (10, $[M - Propyl]^+$), 392 (13, $[M - Propyl-Ethyl]^+$), 196,5 (6, $[M - Propyl-Ethyl]^{2+}$).

$\Delta\varepsilon$ = -3, 3

$\Delta n = 0{,}42$

$\gamma_1$=2035mPa·s

K 126 N 221 I

10) 1-(2,3-Difluor-4-ethoxy-phenylethynyl)-4-(trans-4-n-propyl-cyclohexylethynyl)-naphthalin

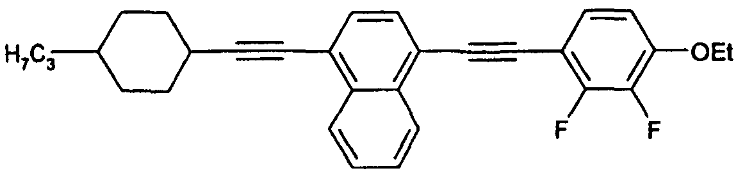

**MS (EI)**: m/z(%) = 456 (100, $M^+$), 360 (27), 331 (10).

$\Delta\varepsilon = -4{,}0$

$\Delta n = 0{,}30$

$\gamma_1$ = 1776 mPa·s

K 123 N 2161 I

11) 1-(4-n-Butyl-phenylethynyl)-4-trans-4-n-propyl-cyclohexylethynyl)-naphthalin

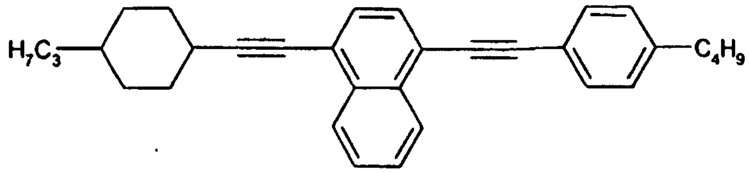

**MS (EI)**: m/z(%) = 432 (100, $M^+$), 336 (29), 291 (17), 279 (13), 265 (11).

$\Delta\varepsilon$ = +1,6
$\Delta n$ = 0,28
$\gamma_1$ = 1749 mPa·s
K 80 N 171 I

12) 1-(3,4,5-Trifluor-phenylethynyl)-4-(4-n-butyl-phenylethynyl)-naphthalin

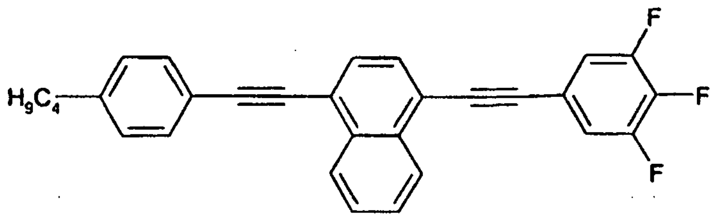

**MS (EI)**: m/z(%) = 438 (100, $M^+$), 395 (72, $[M - Propyl]^+$).

$\Delta\varepsilon$ = +12,2
$\Delta n$ = 0,37
$\gamma_1$ = 964 mPa·s
K 105 N 105.1 I

13) 1-(4-Trifluormethoxy-phenylethynyl)-4-(4-n-butyl-phenylethynyl)-naphthalin

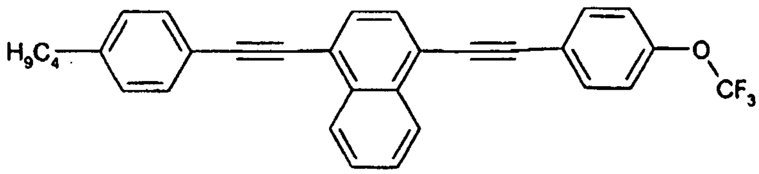

**MS (EI)**: m/z(%) = 468 (100, $M^+$), 425 (58, $[M - Propyl]^+$).

$\Delta\varepsilon$ = +6, 7
$\Delta n$ = 0,38
$\gamma_1$ = 1042 mPa·s
K 96 SmA (82) N 176 I

14) 1-(2-Ethyl-4-n-Butyl-phenylethynyl)-4-(4-n-butyl-phenylethynyl)-naphthalin

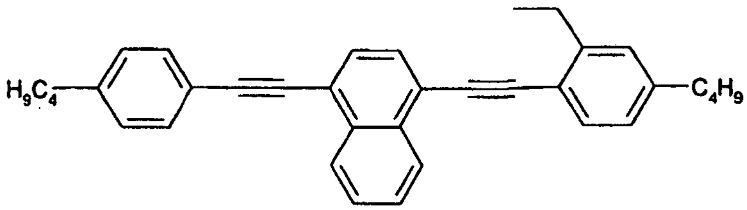

**MS (EI)**: m/z(%) = 468 (100, $M^+$), 425 (17, $[M - Propyl]^+$).

$\Delta\varepsilon$ = +1,1
$\Delta n$ = 0,39
$\gamma_1$ = 1634 mPa·s
Tg -41 K 76 N 105 I

15) 1-(2-Ethyl-4-[1-Hexinyl]-phenylethynyl)-4-(4-n-butyl-phenylethynyl)-naphthalin

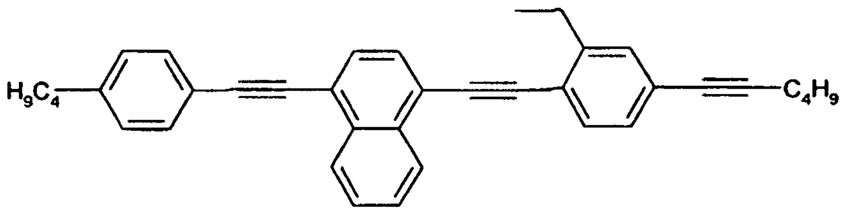

**MS (EI)**: m/z(%) = 492 (100, $M^+$), 449 (8, $[M - Propyl]^+$).

$\Delta\varepsilon$ = +0,8
$\Delta n$ = 0,47
$\gamma_1$ = 6858 mPa·s
Tg-33 K 87 N 103 I

16) 1,4-Bis-(2-Ethyl-4-n-Buyl-Phenylethynyl)-naphthalin

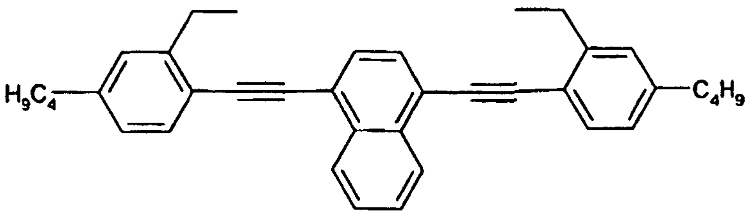

**MS (EI)**: m/z(%) = 496 (100, $M^+$), 453 (10, $[M - Propyl]^+$), 205 (10, $[M\text{-}Propyl\text{-}Propyl]^{2+}$).

$\Delta\varepsilon$ = +0,7

$\Delta n$ = 0,37

$\gamma_1$ = 2394 mPa·s

Tg -45 K 61 N (41) I

17) 1,4-Bis-(4-n-Butyl-phenylethynyl)-anthracen

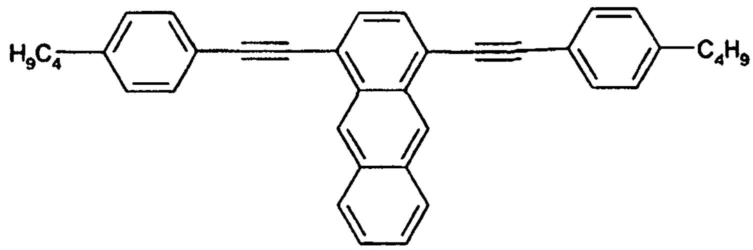

**MS (EI)**: m/z(%) = 490 (100, $M^+$), 447 (21, $[M - Propyl]^+$), 404 (18, $[M-Propyl-Propyl]^+$), 245 (5, $M^{2+}$), 202 (10, $[M - Propyl-Propyl]^{2+}$).

$\Delta\varepsilon$ = +1,1

$\Delta n$ = 0, 39

$\gamma_l$ = 5327 mPa·s

K 132 N (111) I

18) 1-(3,4,5-Trifluor-phenylethynyl)-4-(2-ethyl-4-n-butyl-phenylethyl)-naphthalin

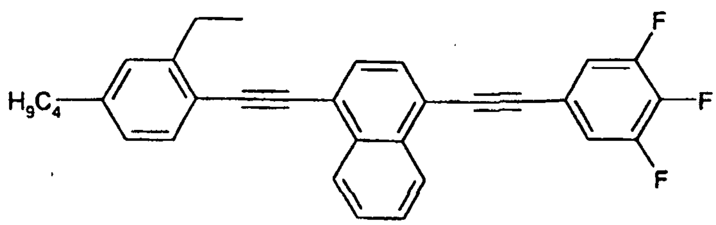

MS (EI): m/z(%) = 466 (100, $M^+$), 423 (72, $[M - Propyl]^+$), 408 (30, $[M-Propyl-Methyl]^+$).

$\Delta\varepsilon$ = +9,6

$\Delta n$ = 0, 36

$\gamma_l$ = 1630 mPa·s

K 122 I

19) 1-(4-Butyl-naphthylethynyl)-4-(4-n-butyl-phenylethynyl)-naphthalin

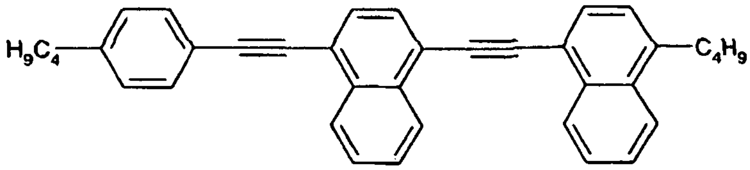

MS (EI): m/z(%) = 490 (100, $M^+$), 447 (66, $[M - Propyl]^+$), 404 (28, $[M-Propyl-Propyl]^+$), 202 (18, $[M - Propyl-Propyl]^{2+}$).

$\Delta\varepsilon$ = +0,9

$\Delta n$ = 0,40

$\gamma_1$ = 5261 mPa·s

K 114 N (110) I

20) 1,4-Bis [4-(4'-Butyl-phenylethynyl)-naphthylethynyl]-benzol

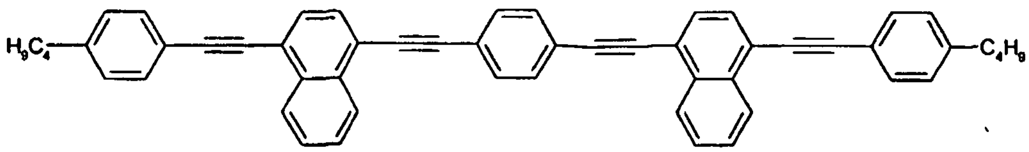

**MS (EI)**: m/z(%) = 690 (100, $M^+$), 647 (13, $[M - Propyl]^+$), 604 (7, $[M-Propyl-Propyl]^+$), 302 (25, $[M - Propyl-Propyl]^{2+}$).

K 187 N 3101 I

22) 1-(3,4,5-Trifluor-phenylethynyl)-4-(2,6-Difluor-4-n-butyl-phenylethynyl)-naphthalin

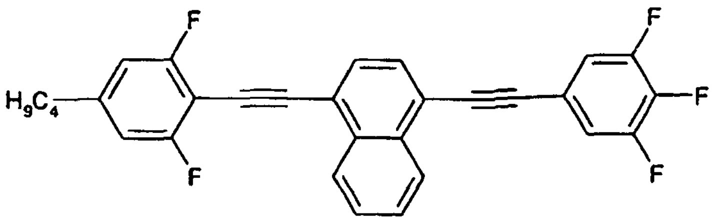

MS (EI): m/z(%) = 474 (100, $M^+$), 431 (55, $[M - Propyl]^+$).

$\Delta\varepsilon$ = +16,8

$\Delta n$ = 0,37

K 126 N (122) I

23) 1-(4-Trifluormethyl-3,5-difluor-phenylethynyl)-4-(2,6-Difluor-4-n-butyl)-phenylethynyl)-naphthalin

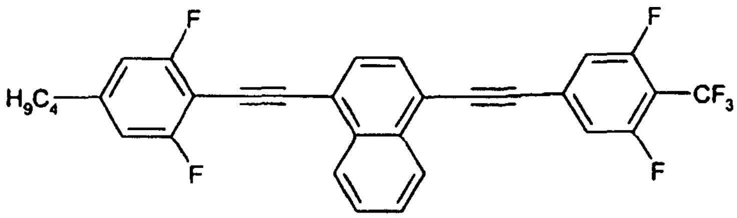

**MS (EI)**: m/z(%) = 524 (100, $M^+$), 481 (69, $[M - Propyl]^+$).

$\Delta\varepsilon$ = +24,9
$\Delta n$ = 0,36
$\gamma_1$ = 759 mPa·s
K 136 I

24) 1-(4-Trifluormethoxy-3,5-difluor-phenylethynyl)-4-(2,6-Difluor-4-n-butyl-phenylethynyl)-naphthalin

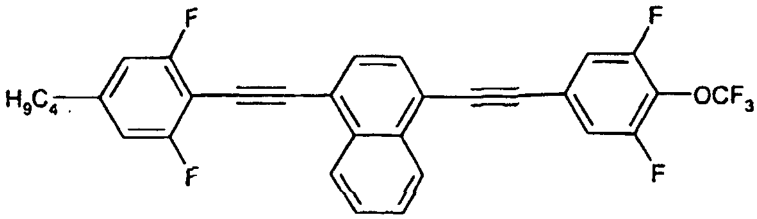

MS (EI): m/z(%) = 540 (100, $M^+$), 497 (43, $[M - Propyl]^+$), 428 (11, $[M\text{-}Propyl\text{-}Trifluormethoxy]^+$).

K 127 N (125) I

25) 1-(4-Cyano-phenylethynyl)-4-(4-n-propyl-phenylethynyl)-naphthalin

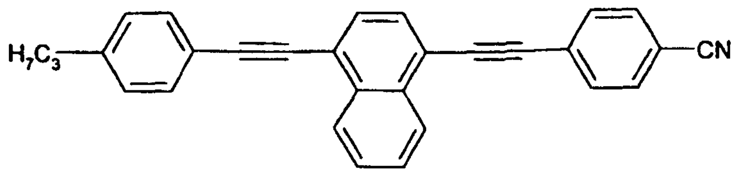

MS (EI): m/z(%) = 395 (100, $M^+$), 366 (56, $[M - Ethyl]^+$), 183 (12, $[M\text{-}Ethyl]^{2}+$).

K 150 N 250 I

26) 1-(4-Fluor-naphthylethynyl)-4-(4-n-butyl-phenylethynyl)-benzol

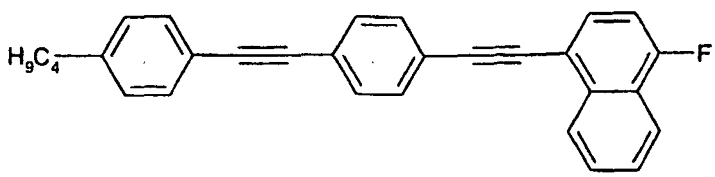

**MS (EI)**: m/z(%) = 402 (100, $M^+$), 359 (62, $[M - Propyl]^+$), 179,5 (18, $[M\text{-}Propyl]^{2+}$).

$\Delta\varepsilon$ = +5,5
$\Delta n$ = 0,39
$\gamma_1$ = 1140 mPa·s
K 123 N 133 I

27) 1-(4-n-Butyl-naphthylethynyl)-4-(4-n-buyl-phenylethynyl)-benzol

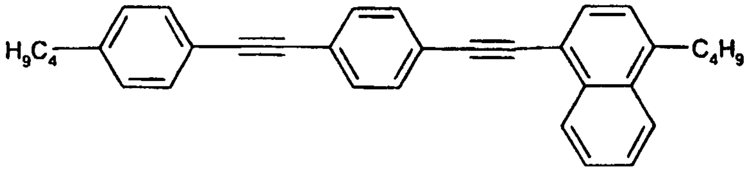

**MS (EI)**: m/z(%) = 440 (100, $M^+$), 397 (52, $[M - Propyl]^+$), 354 (20, $[M\text{-}Propyl\text{-}Propyl]^+$), 177 (22, $[M - Propyl\text{-}Propyl]^{2+}$).

$\Delta\varepsilon$ = +2,0
$\Delta n$ = 0,38
$\gamma_1$ = 1438 mPa·s
K 105 N 1371 I

28) 1-(4-n-Propyl-naphthylethynyl)-4-(4-n-butyl-phenylethynyl)-benzol

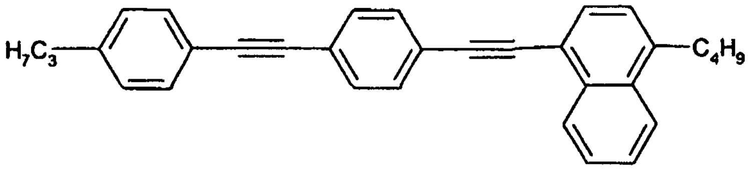

**MS (EI)**: m/z(%) =426 (100, $M^+$), 383 (52, $[M - Propyl]^+$), 354 (18, $[M\text{-}Propyl\text{-}Ethyl]^+$), 177 (22, $[M - Propyl\text{-}Ethyl]^{2+}$).

$\Delta\varepsilon$ = +1,9
$\Delta n$ = 0, 39
$\gamma_1$ = 1820 mPa·s
K 109 N 154 I

Mischungsbeispiel 1

Es wird eine Flüssigkristallmischung M-1 mit der Zusammensetzung und den Eigenschaften wie in der folgenden Tabelle angegeben hergestellt. Die Komponente (1) ist die Verbindung aus Synthesebeispiel 4).

| Zusammensetzung | | | Physikalische Eigenschaften | | |
|---|---|---|---|---|---|
| Verbindung | | | T(N,I) | = 103 | °C |
| Nr. | Abkürzung | | | | |
| 1 | BCH-3F.F | 10,8 % | | | |
| 2 | BCH-5F.F | 9,00 % | $\Delta n$ (20°C, 589,3 nm) = | 0,130 | |
| 3 | ECCP-30CF3 | 4,50 % | | | |
| 4 | ECCP-50CF3 | 4,50 % | | | |

(fortgesetzt)

| Zusammensetzung | | | Physikalische Eigenschaften | | |
|---|---|---|---|---|---|
| Verbindung | | | T(N,I) | = 103 | °C |
| Nr. | Abkürzung | | | | |
| 5 | CBC-33F | 1,80 % | $\Delta\varepsilon$ (20°C, 1 kHz) | = 5,0 | |
| 6 | CBC-53F | 1,80 % | | | |
| 7 | CBC-55F | 1,80 % | $\gamma_1$ (20°C) | = 176 | mPa · s |
| 8 | PCH-6F | 7,20 % | | | |
| 9 | PCH-7F | 5,40 % | | | |
| 10 | CCP-20CF3 | 7,20 % | | | |
| 11 | CCP-30CF3 | 10,8 % | | | |
| 12 | CCP-40CF3 | 6,30 % | | | |
| 13 | CCP-50CF3 | 9,90 % | | | |
| 14 | PCH-5F | 9,00 % | | | |
| 15 | (1) | 10.0 % | | | |
| Σ | | 100,0 % | | | |

Diese flüssigkristalline Mischung wird für Anwendungen im Mikrowellenbereich, insbesondere für einen Phasenschieber ('phased array') verwendet.

Zum Vergleich wird eine Mischung C-1 ohne die Komponente (1) aus den Verbindungen Nr. 1-14 der M-1 hergestellt, wobei die Verbindungen Nr. 1-14 in den gleichen relativen Mengen enthalten sind.

Tabelle: Eigenschaften der Mischung M-1 und C-2 bei 19 GHz (20°C)

| Mis | $\varepsilon_{r,\|\|}$ | $\varepsilon_{r,\perp}$ | T | tan $\delta_{\varepsilon, r,\|\|}$ | tan $\delta_{\varepsilon, \rho,\perp}$ | $\eta$ |
|---|---|---|---|---|---|---|
| M-1 | 2,60 | 2, 34 | 0,101 | 0,0041 | 0,0127 | 7,95 |
| C-1 | 2,49 | 2,30 | 0,079 | 0,0048 | 0,0139 | 5,70 |

Die Steuerbarkeit $\tau$ und die Materialgüte $\eta$ sind gegenüber der Vergleichsmischung C-1 verbessert.

Weitere Kombinationen der Ausführungsformen und Varianten der Erfindung gemäß der Beschreibung ergeben sich auch aus den folgenden Ansprüchen.

**Patentansprüche**

1. Verbindungen der Formel I

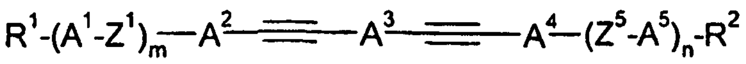

I

worin

$A^{1-5}$ unabhängig voneinander

a) einen Rest der Formeln

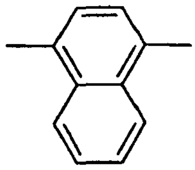 oder 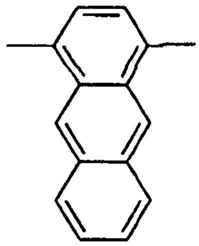 ,

b) 1,4-Phenylen, worin ein oder mehrere CH-Gruppen durch N ersetzt sein können, oder

c) trans-1,4-Cyclohexylen oder Cyclohexenylen, worin auch eine oder zwei nicht benachbarte $CH_2$-Gruppen durch -O- und/oder -S- ersetzt sein können, und worin H durch F ersetzt sein kann,

d) einen Rest aus der Gruppe 1,4-Bicyclo(2,2,2)-octylen, Cylcobut-1,3-diyl, Spiro[3.3]heptan-2,6-diyl, Thiophen-2,5-diyl, Thiophen-2,4-diyl, Furan-2,5-diyl, Furan-2,4-diyl,

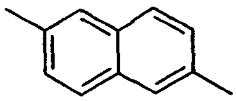 oder 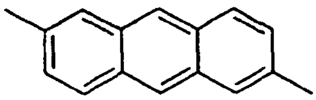 ,

und worin in den Gruppen a), b), c) und d)
auch ein oder mehrere H-Atome gegen Br, Cl, F, CN, -NCS, -SCN, $SF_5$, $C_1$-$C_{10}$ Alkyl, $C_1$-$C_{10}$ Alkoxy oder eine ein- oder mehrfach fluorierte $C_1$-$C_{10}$ Alkyl- oder Alkoxygruppe ersetzt sein können,
und wobei

mindestens ein vorhandener Rest aus $A^1$ bis $A^5$ einen Rest nach a) darstellt,

$R^1$ und $R^2$ jeweils unabhängig voneinander einen halogenierten oder unsubstituierten Alkylrest mit 1 bis 15 C-Atomen, wobei in diesen Resten auch eine oder mehrere $CH_2$-Gruppen jeweils unabhängig voneinander durch -C≡C-, -CH=CH-, -CF=CF-, -CF=CH-, -CH=CF-, -(CO)O-, -O(CO)-, -(CO)-, -O- oder -S- so ersetzt sein können, dass O- oder S-Atome nicht direkt miteinander verknüpft sind, F, Cl, Br, CN, $CF_3$, $OCF_3$, SCN, NCS oder $SF_5$,

$Z^1$, $Z^5$ unabhängig voneinander, eine Einfachbindung, -C≡C-, -CH=CH-, $-CH_2O-$, -(CO)O-, $-CF_2O-$, $-CF_2CF_2-$, $-CH_2CF_2-$, $-CH_2CH_2-$, $-(CH_2)_4-$, -CH=CF- oder -CF=CF-, wobei asymmetrische Brücken nach beiden Seiten orientiert sein können, und

m, n unabhängig voneinander 0, 1 oder 2

bedeuten,
wobei Verbindungen der Formel I-X-1,

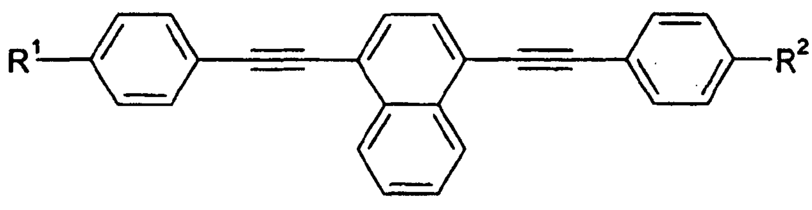

I-X-1

worin
$R^1$ und $R^2$ jeweils gleichzeitig $CH_3$, n-$C_6H_{11}$, $CF_3$, F oder $OCH_3$ sind, oder
$R^1$ oder $R^2$ ein tert-Butyl und die andere Gruppe -CN bedeuten, und die Verbindung der Formel I-X-2

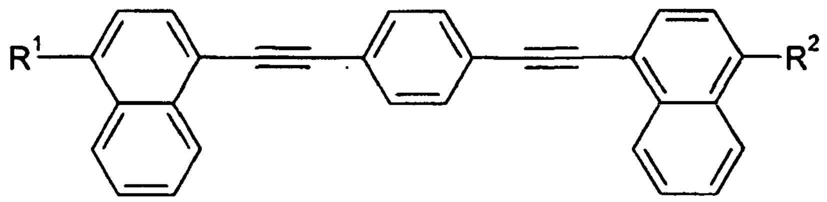

I-X-2

worin
$R^1$ und $R^2$ gleichzeitig n-$C_4H_9$ bedeuten,
ausgeschlossen sind.

2. Verbindungen nach Anspruch 1, **dadurch gekennzeichnet, dass** mindestens eine Gruppe aus $A^2$, $A^3$ und $A^4$ einen optional substituierten 1,4-Naphthylenrest oder 1,4-Anthracenylenrest bedeutet.

3. Verbindungen nach Anspruch 1 oder 2, **dadurch gekennzeichnet, dass** die Verbindungen der Formel I einen oder zwei optional substituierte 1,4-Naphthylenreste oder 1,4-Anthracenylenreste enthalten.

4. Verbindungen nach einem oder mehreren der Ansprüche 1 bis 3, **dadurch gekennzeichnet, dass** m und n 0 sind.

5. Flüssigkristallmedium, **dadurch gekennzeichnet, dass** es eine oder mehrere Verbindungen der Formel I

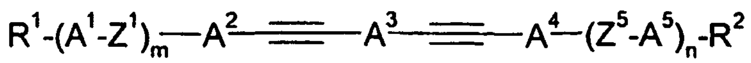

I

worin

$A^{1-5}$ unabhängig voneinander

a) einen Rest der Formeln

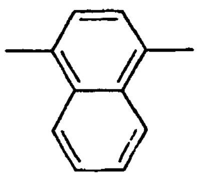

oder

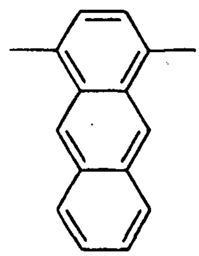

b) 1,4-Phenylen, worin ein oder mehrere CH-Gruppen durch N ersetzt sein können,
oder
c) trans-1,4-Cyclohexylen oder Cyclohexenylen, worin auch eine oder zwei nicht benachbarte $CH_2$-Gruppen durch -O- und/oder -S- ersetzt sein können, und worin H durch F ersetzt sein kann,
d) einen Rest aus der Gruppe 1,4-Bicyclo(2,2,2)-octylen, Cylcobut-1,3-diyl, Spiro[3.3]heptan-2,6-diyl, Thiophen-2,5-diyl, Thiophen-2,4-diyl, Furan-2,5-diyl, Furan-2,4-diyl,

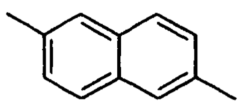

oder

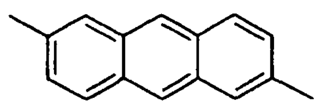

,

und worin in den Gruppen a), b), c) und d)
auch ein oder mehrere H-Atome gegen Br, Cl, F, CN, -NCS, -SCN, $SF_5$, $C_1$-$C_{10}$ Alkyl, $C_1$-$C_{10}$ Alkoxy oder eine ein- oder mehrfach fluorierte $C_1$-$C_{10}$ Alkyl- oder Alkoxygruppe ersetzt sein können, und wobei

mindestens ein Rest aus $A^1$ bis $A^5$ einen Rest nach a) darstellt,

$R^1$ und $R^2$ jeweils unabhängig voneinander einen halogenierten oder unsubstituierten Alkylrest mit 1 bis 15 C-Atomen, wobei in diesen Resten auch eine oder mehrere $CH_2$-Gruppen jeweils unabhängig voneinander durch -C≡C-, -CH=CH-, -CF=CF-, -CF=CH-, -CH=CF-, -(CO)O-, -O(CO)-, -(CO)- oder -O- so ersetzt sein können, dass O-Atome nicht direkt miteinander verknüpft sind, bedeuten, F, Cl, Br, CN, $CF_3$, $OCF_3$, SCN, NCS oder $SF_5$,
$Z^1$, $Z^5$ unabhängig voneinander, eine Einfachbindung, -C≡C-, -CH=CH-, $-CH_2O-$, -(CO)O-, $-CF_2O-$, $-CF_2CF_2-$, $-CH_2CF_2-$, $-CH_2CH_2-$, $-(CH_2)_4-$, -CH=CF- oder -CF=CF-, wobei asymmetrische Brücken nach beiden Seiten orientiert sein können,und
m, n unabhängig voneinander 0, 1 oder 2

bedeuten,
enthält.

6. Flüssigkristallmedium nach Anspruch 5, **dadurch gekennzeichnet, dass** es zusätzlich eine oder mehrere Verbindungen ausgewählt aus den Verbindungen der Formel II enthält:

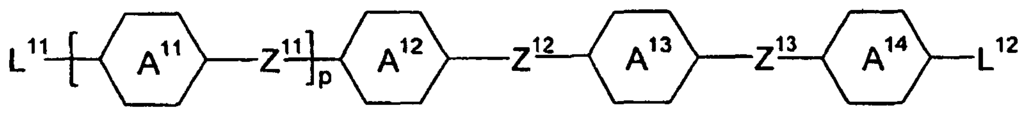
II

worin

$L^{11}$ $R^{11}$ oder $X^{11}$,
$L^{12}$ $R^{12}$ oder $X^{12}$,
$R^{11}$ und $R^{12}$ unabhängig voneinander unfluoriertes Alkyl oder unfluoriertes Alkoxy mit 1 bis 17 C-Atomen oder unfluoriertes Alkenyl, unfluoriertes Alkenyloxy, unfluoriertes Alkinyl oder unfluoriertes Alkoxyalkyl mit 2 bis 15 C-Atomen bedeuten,
$X^{11}$ und $X^{12}$ unabhängig voneinander F, Cl, Br, -CN, -NCS, -SCN, $-SF_5$, fluoriertes Alkyl oder fluoriertes Alkoxy mit 1 bis 7 C-Atomen oder fluoriertes Alkenyl, fluoriertes Alkenyloxy oder fluoriertes Alkoxyalkyl mit 2 bis 7 C-Atomen bedeuten, und
p 0 oder 1
$Z^{11}$ bis $Z^{13}$ unabhängig voneinander *trans*--CH=CH-, *trans*--CF=CF-, -C≡C- oder eine Einfachbindung bedeuten, und

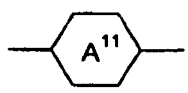
bis
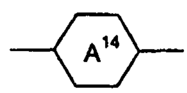

unabhängig voneinander

a) 1,4-Phenylen, worin ein oder mehrere, bevorzugt ein bis zwei CH-Gruppen durch N ersetzt sein können,
b) trans-1,4-Cyclohexylen oder Cyclohexenylen, worin auch eine oder zwei nicht benachbarte $CH_2$-Gruppen durch -O- und/oder -S- ersetzt sein können, und worin H durch F ersetzt sein kann,

und worin in den Gruppen a) und b)
auch ein oder mehrere H-Atome gegen Br, Cl, F, CN, -NCS, -SCN, $SF_5$, $C_1$-$C_{10}$ Alkyl, $C_1$-$C_{10}$ Alkoxy, eine ein- oder mehrfach fluorierte $C_1$-$C_{10}$ Alkyl- oder Alkoxygruppe oder eine $C_{3-6}$ Cycloalkylgruppe ersetzt sein können,

bedeuten.

7. Flüssigkristallmedium nach Anspruch 5 oder 6, **dadurch gekennzeichnet, dass** die Konzentration der Verbindungen der Formel I im Medium im Bereich von insgesamt 5 % bis 95 % liegt.

8. Verwendung der Verbindungen der Formel I nach einem oder mehreren der Ansprüche 5 oder 1 bis 4 in einer flüssigkristallinen Mischung.

9. Verwendung der Verbindungen der Formel I nach einem oder mehreren der Ansprüche 5 oder 1 bis 4 in einem Bauteil für die Hochfrequenztechnik.

10. Verfahren zur Herstellung eines Flüssigkristallmediums nach einem oder mehreren der Ansprüche 5 bis 7, **dadurch gekennzeichnet, dass** eine oder mehrere Verbindungen der Formel I, wie in Anspruch 5 angegeben, mit einer oder mehreren Verbindungen ausgewählt aus den Verbindungen der Formel II, wie in Anspruch 6 angegeben und optional mit einer oder mehreren weiteren Verbindungen und optional mit einem oder mehreren Additiven gemischt wird.

11. Bauteil für die Hochfrequenztechnik, **dadurch gekennzeichnet, dass** es ein Flüssigkristallmedium nach einem oder mehreren der Ansprüche 5 bis 7 enthält.

12. Bauteil nach Anspruch 11, **dadurch gekennzeichnet, dass** es sich um einen oder mehrere funktionell verbundene Phasenschieber handelt.

13. Verwendung eines Flüssigkristallmediums nach einem oder mehreren der Ansprüche 5 bis 7 in einem Bauteil für die Hochfrequenztechnik.

14. Phasengesteuerte Gruppenantenne, **dadurch gekennzeichnet, dass** sie ein oder mehrere Bauteile nach einem oder mehreren der Ansprüche 11 oder 12 enthält.

## Claims

1. Compounds of the formula I

$$R^1\text{-}(A^1\text{-}Z^1)_m\text{—}A^2\text{—}\equiv\text{—}A^3\text{—}\equiv\text{—}A^4\text{—}(Z^5\text{-}A^5)_n\text{-}R^2 \qquad \text{I}$$

in which

$A^{1-5}$, independently of one another, denote

a) a radical of the formula

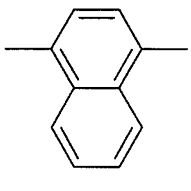

or

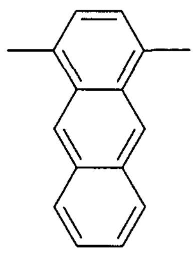

,

b) 1,4-phenylene, in which one or more CH groups may be replaced by N,
c) trans-1,4-cyclohexylene or cyclohexenylene, in which, in addition, one or two non-adjacent $CH_2$ groups may be replaced by -O- and/or -S- and in which H may be replaced by F,
or
d) a radical from the group 1,4-bicyclo[2.2.2]octylene, cyclobut-1,3-diyl, spiro[3.3]heptane-2,6-diyl, thiophene-2,5-diyl, thiophene-2,4-diyl, furan-2,5-diyl, furan-2,4-diyl,

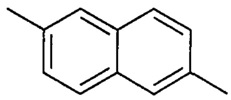 or 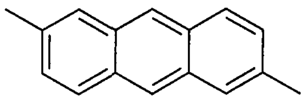 ,

and in which, in groups a), b), c) and d), one or more H atoms may also be replaced by Br, Cl, F, CN, -NCS, -SCN, $SF_5$, $C_1$-$C_{10}$ alkyl, $C_1$-$C_{10}$ alkoxy or a mono- or polyfluorinated $C_1$-$C_{10}$ alkyl or alkoxy group,

and where at least one radical present from $A^1$ to $A^5$ represents a radical according to a),

$R^1$ and $R^2$ each, independently of one another, denote a halogenated or unsubstituted alkyl radical having 1 to 15 C atoms, where, in addition, one or more $CH_2$ groups in these radicals may each be replaced, independently of one another, by -C≡C-, -CH=CH-, -CF=CF-, -CF=CH-, -CH=CF-, -(CO)O-, -O(CO)-, -(CO)-, -O- or -S- in such a way that O or S atoms are not linked directly to one another, F, Cl, Br, CN, $CF_3$, $OCF_3$, SCN, NCS or $SF_5$,

$Z^1$, $Z^5$, independently of one another, denote a single bond, -C≡C-, -CH=CH-, -$CH_2$O-, -(CO)O-, -$CF_2$O-, -$CF_2CF_2$-, -$CH_2CF_2$-, -$CH_2CH_2$-, -$(CH_2)_4$-, -CH=CF- or -CF=CF-, where asymmetrical bridges may be oriented to both sides, and

m, n, independently of one another, denote 0, 1 or 2,

where compounds of the formula I-X-1,

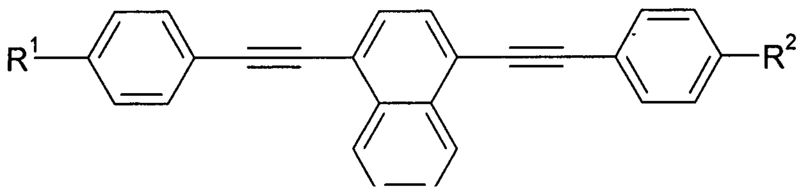

I-X-1

in which
$R^1$ and $R^2$ are each simultaneously $CH_3$, n-$C_6H_{11}$, $CF_3$, F or $OCH_3$, or $R^1$ or $R^2$ denotes tert-butyl and the other group denotes -CN,
and the compound of the formula I-X-2

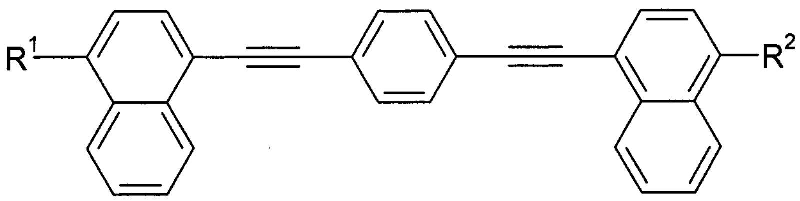

I-X-2

in which
$R^1$ and $R^2$ simultaneously denote n-$C_4H_9$, are excluded.

2. Compounds according to Claim 1, **characterised in that** at least one group from $A^2$, $A^3$ and $A^4$ denotes an optionally substituted 1,4-naphthylene radical or 1,4-anthracenylene radical.

3. Compounds according to Claim 1 or 2, **characterised in that** the compounds of the formula I contain one or two optionally substituted 1,4-naphthylene radicals or 1,4-anthracenylene radicals.

4. Compounds according to one or more of Claims 1 to 3, **characterised in that** m and n are 0.

5. Liquid-crystal medium, **characterised in that** it comprises one or more compounds of the formula I

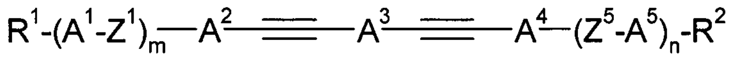

I

in which

$A^{1-5}$, independently of one another, denote

a) a radical of the formula

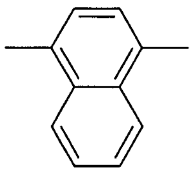 or 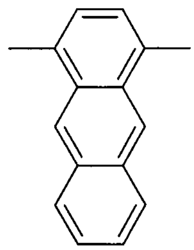 ,

b) 1,4-phenylene, in which one or more CH groups may be replaced by N,
c) trans-1,4-cyclohexylene or cyclohexenylene, in which, in addition, one or two non-adjacent $CH_2$ groups may be replaced by -O- and/or -S- and in which H may be replaced by F,
or
d) a radical from the group 1,4-bicyclo[2.2.2]octylene, cyclobut-1,3-diyl, spiro[3.3]heptane-2,6-diyl, thiophene-2,5-diyl, thiophene-2,4-diyl, furan-2,5-diyl, furan-2,4-diyl,

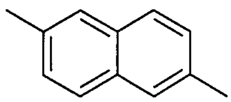 or 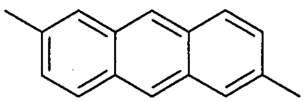 ,

and in which, in groups a), b), c) and d),
one or more H atoms may also be replaced by Br, Cl, F, CN, -NCS, -SCN, $SF_5$, $C_1$-$C_{10}$ alkyl, $C_1$-$C_{10}$ alkoxy or a mono- or polyfluorinated $C_1$-$C_{10}$ alkyl or alkoxy group,

and where
at least one radical from $A^1$ to $A^5$ represents a radical according to a),

$R^1$ and $R^2$ each, independently of one another, denote a halogenated or unsubstituted alkyl radical having 1 to 15 C atoms, where, in addition, one or more $CH_2$ groups in these radicals may each be replaced, independently of one another, by -C≡C-, -CH=CH-, -CF=CF-, -CF=CH-, -CH=CF-, -(CO)O-, -O(CO)-, -(CO)- or -O- in such a way that O atoms are not linked directly to one another, F, Cl, Br, CN, $CF_3$, $OCF_3$, SCN, NCS or $SF_5$,
$Z^1$, $Z^5$, independently of one another, denote a single bond, -C≡C-, -CH=CH-, $-CH_2O-$, -(CO)O-, $-CF_2O-$, $-CF_2CF_2-$, $-CH_2CF_2-$, $-CH_2CH_2-$, $-(CH_2)_4-$, -CH=CF- or -CF=CF-, where asymmetrical bridges may be oriented to both sides, and
m, n, independently of one another, denote 0, 1 or 2.

**6.** Liquid-crystal medium according to Claim 5, **characterised in that** it additionally comprises one or more compounds selected from the compounds of the formula II:

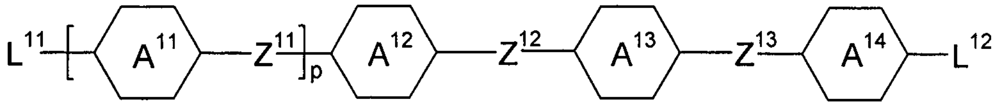

II

in which

$L^{11}$ denotes $R^{11}$ or $X^{11}$,
$L^{12}$ denotes $R^{12}$ or $X^{12}$,
$R^{11}$ and $R^{12}$, independently of one another, denote unfluorinated alkyl or unfluorinated alkoxy having 1 to 17 C atoms or unfluorinated alkenyl, unfluorinated alkenyloxy, unfluorinated alkynyl or unfluorinated alkoxyalkyl having 2 to 15 C atoms,
$X^{11}$ and $X^{12}$, independently of one another, denote F, Cl, Br, -CN, -NCS, -SCN, $-SF_5$, fluorinated alkyl or fluorinated alkoxy having 1 to 7 C atoms or fluorinated alkenyl, fluorinated alkenyloxy or fluorinated alkoxyalkyl having 2 to 7 C atoms, and
p denotes 0 or 1,
$Z^{11}$ to $Z^{13}$, independently of one another, denote trans-CH=CH-, trans-CF=CF-, -C=C- or a single bond, and

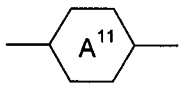
to
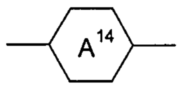
,

independently of one another, denote

a) 1,4-phenylene, in which one or more, preferably one or two, CH groups may be replaced by N,
b) trans-1,4-cyclohexylene or cyclohexenylene, in which, in addition, one or two non-adjacent $CH_2$ groups may be replaced by -O- and/or -S- and in which H may be replaced by F,

and in which, in groups a) and b),
one or more H atoms may also be replaced by Br, Cl, F, CN, -NCS, -SCN, $SF_5$, $C_1$-$C_{10}$ alkyl, $C_1$-$C_{10}$ alkoxy, a mono- or polyfluorinated $C_1$-$C_{10}$ alkyl or alkoxy group or a $C_3$-$C_6$ cycloalkyl group.

**7.** Liquid-crystal medium according to Claim 5 or 6, **characterised in that** the concentration of the compounds of the formula I in the medium is in the range from in total 5% to 95%.

**8.** Use of the compounds of the formula I according to one or more of Claims 5 or 1 to 4 in a liquid-crystalline mixture.

**9.** Use of the compounds of the formula I according to one or more of Claims 5 or 1 to 4 in a component for high-frequency technology.

**10.** Process for the preparation of a liquid-crystal medium according to one or more of Claims 5 to 7, **characterised in that** one or more compounds of the formula I, as indicated in Claim 5, are mixed with one or more compounds selected from the compounds of the formula II, as indicated in Claim 6, and optionally with one or more further compounds and optionally with one or more additives.

**11.** Component for high-frequency technology, **characterised in that** it comprises a liquid-crystal medium according to one or more of Claims 5 to 7.

**12.** Component according to Claim 11, **characterised in that** it is one or more functionally connected phase shifters.

**13.** Use of a liquid-crystal medium according to one or more of Claims 5 to 7 in a component for high-frequency technology.

**14.** Phase-controlled group antenna, **characterised in that** it comprises one or more components according to one or more of Claims 11 or 12.

### Revendications

**1.** Composés de la formule I

$$R^1\text{-}(A^1\text{-}Z^1)_m\text{—}A^2\equiv A^3\equiv A^4\text{—}(Z^5\text{-}A^5)_n\text{-}R^2 \qquad \text{I}$$

dans laquelle

$A^{1-5}$ représentent, indépendamment les uns des autres,

a) un radical de la formule

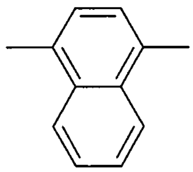

ou

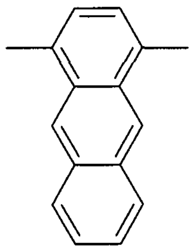

,

b) 1,4-phénylène, où un ou plusieurs groupes CH peut/peuvent être remplacé(s) par N,

c) trans-1,4-cyclohexylène ou cyclohexénylène, où, en outre, un ou deux groupes $CH_2$ non adjacents peut/peuvent être remplacé(s) par -O- et/ou -S- et où H peut être remplacé par F, ou

d) un radical pris parmi le groupe 1,4-bicyclo[2.2.2]octylène, cyclobut-1,3-diyle, spiro[3.3]heptane-2,6-diyle, thiophène-2,5-diyle, thiophène-2,4-diyle, furan-2,5-diyle, furan-2,4-diyle,

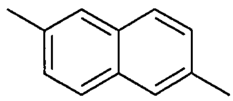

ou

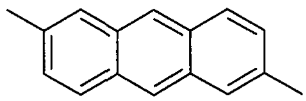

,

et où, dans les groupes a), b), c) et d),

un ou plusieurs atomes de H peut/peuvent également être remplacé(s) par Br, Cl, F, CN, -NCS, -SCN, $SF_5$, $C_1$-$C_{10}$ alkyle, $C_1$-$C_{10}$ alcoxy ou un groupe $C_1$-$C_{10}$ alkyle ou alcoxy mono- ou polyfluoré,

et où

au moins un radical présent pris parmi $A^1$ à $A^5$ représente un radical conformément à a),

$R^1$ et $R^2$ représentent, chacun indépendamment de l'autre, un radical alkyle halogéné ou non substitué comportant de 1 à 15 atomes de C, où, en outre, un ou plusieurs groupes $CH_2$ dans ces radicaux peuvent chacun être remplacés, indépendamment les uns des autres, par -C≡C-, -CH=CH-, -CF=CF-, -CF=CH-, -CH=CF-, -(CO)O-, -O(CO)-, -(CO)-, -O- ou -S- de telle sorte que des atomes de O ou de S ne soient pas liés directement les uns aux autres, F, Cl, Br, CN, $CF_3$, $OCF_3$, SCN, NCS ou $SF_5$,

$Z^1$, $Z^5$ représentent, indépendamment l'un de l'autre, une liaison simple, -C≡C-, -CH=CH-, $-CH_2O-$, -(CO)O-, $-CF_2O-$, $-CF_2CF_2-$, $-CH_2CF_2-$, $-CH_2CH_2-$, $-(CH_2)_4-$, -CH=CF- ou -CF=CF-, où des ponts asymétriques peuvent être orientés sur les deux côtés, et

m, n représentent, indépendamment l'un de l'autre, 0, 1 ou 2,

où des composés de la formule I-X-1,

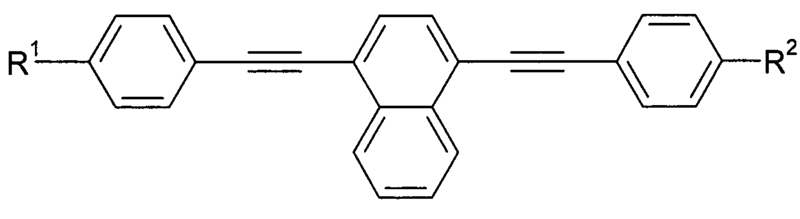

I-X-1

dans laquelle
$R^1$ et $R^2$ sont chacun simultanément $CH_3$, n-$C_6H_{11}$, $CF_3$, F ou $OCH_3$, ou
$R^1$ ou $R^2$ représente tert-butyle et l'autre groupe représente -CN,
et le composé de la formule I-X-2

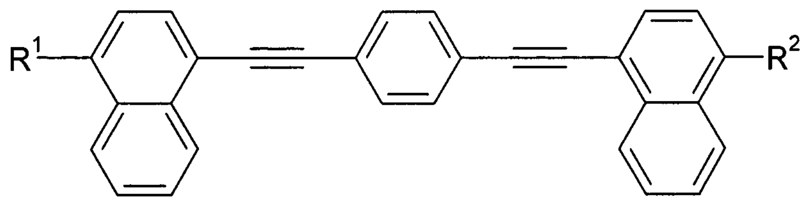

I-X-2

dans laquelle
$R^1$ et $R^2$ représentent simultanément n-$C_4H_9$,
sont exclus.

**2.** Composés selon la revendication 1, **caractérisés en ce qu'**au moins un groupe pris parmi $A^2$, $A^3$ et $A^4$ représente un radical 1,4-naphtylène ou un radical 1,4-anthracénylène en option substitué.

**3.** Composés selon la revendication 1 ou 2, **caractérisés en ce que** les composés de la formule I contiennent un ou deux radicaux 1,4-naphtylène ou radicaux 1,4-anthracénylène en option substitués.

**4.** Composés selon une ou plusieurs des revendications 1 à 3, **caractérisés en ce que** m et n sont 0.

**5.** Milieu cristallin liquide, **caractérisé en ce qu'**il comprend un ou plusieurs composés de la formule I

$$R^1-(A^1-Z^1)_m-A^2\equiv A^3\equiv A^4-(Z^5-A^5)_n-R^2 \qquad I$$

dans laquelle

$A^{1-5}$ représentent, indépendamment les uns des autres,

a) un radical de la formule

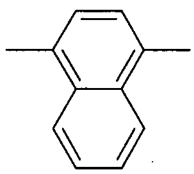 ou 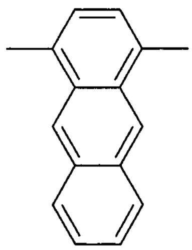 ,

b) 1,4-phénylène, où un ou plusieurs groupes CH peut/peuvent être remplacé(s) par N,
c) trans-1,4-cyclohexylène ou cyclohexénylène, où, en outre, un ou deux groupes $CH_2$ non adjacents peut/peuvent être remplacé(s) par -O- et/ou -S- et où H peut être remplacé par F, ou
d) un radical pris parmi le groupe 1,4-bicyclo[2.2.2]octylène, cyclobut-1,3-diyle, spiro[3.3]heptane-2,6-diyle, thiophène-2,5-diyle, thiophène-2,4-diyle, furan-2,5-diyle, furan-2,4-diyle,

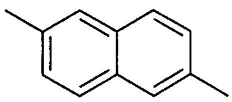 ou 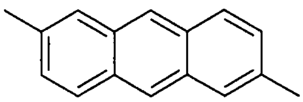 ,

et où, dans les groupes a), b), c) et d),
un ou plusieurs atomes de H peut/peuvent être remplacé(s) par Br, Cl, F, CN, -NCS, -SCN, $SF_5$, $C_1$-$C_{10}$ alkyle, $C_1$-$C_{10}$ alcoxy ou un groupe $C_1$-$C_{10}$ alkyle ou alcoxy mono- ou polyfluoré,

et où
au moins un radical pris parmi $A^1$ à $A^5$ représente un radical conformément à a),

$R^1$ et $R^2$ représentent, chacun indépendamment de l'autre, un radical alkyle halogéné ou non substitué comportant 1 à 15 atomes de C, où, en outre, un ou plusieurs groupes $CH_2$ dans ces radicaux peuvent chacun être remplacés, indépendamment les uns des autres, par -C≡C-, -CH=CH-, -CF=CF-, -CF=CH-, -CH=CF-, -(CO)O-, -O(CO)-, -(CO)- ou -O- de telle sorte que des atomes de O ne soient pas liés directement les uns aux autres, F, Cl, Br, CN, $CF_3$, $OCF_3$, SCN, NCS ou $SF_5$,
$Z^1$, $Z^5$ représentent, indépendamment l'un de l'autre, une liaison simple, -C≡C-, -CH=CH-, $-CH_2O-$, -(CO)O-, $-CF_2O-$, $-CF_2CF_2-$, $-CH_2CF_2-$, $-CH_2CH_2-$, $-(CH_2)_4-$, -CH=CF- ou -CF=CF-, où des ponts asymétriques peuvent être orientés sur les deux côtés, et
m, n représentent, indépendamment l'un de l'autre, 0, 1 ou 2.

**6.** Milieu cristallin liquide selon la revendication 5, **caractérisé en ce qu'**il comprend additionnellement un ou plusieurs composés choisis parmi les composés de la formule II :

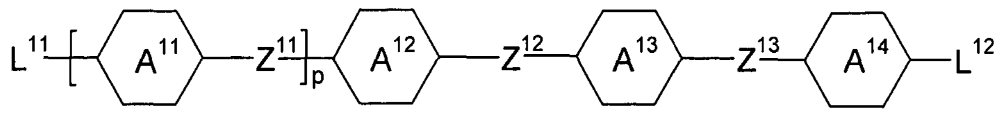
II

dans laquelle

$L^{11}$ représente $R^{11}$ ou $X^{11}$,
$L^{12}$ représente $R^{12}$ ou $X^{12}$,
$R^{11}$ et $R^{12}$ représentent, indépendamment l'un de l'autre, alkyle non fluoré ou alcoxy non fluoré comportant de 1 à 17 atomes de C ou alkényle non fluoré, alkényloxy non fluoré, alkynyle non fluoré ou alcoxyalkyle non fluoré comportant de 2 à 15 atomes de C,
$X^{11}$ et $X^{12}$ représentent, indépendamment l'un de l'autre, F, Cl, Br, -CN, -NCS, -SCN, $-SF_5$, alkyle fluoré ou alcoxy fluoré comportant de 1 à 7 atomes de C ou alkényle fluoré, alkényloxy fluoré ou alcoxyalkyle fluoré comportant de 2 à 7 atomes de C, et
p représente 0 ou 1,
$Z^{11}$ à $Z^{13}$ représentent, indépendamment les uns des autres, *trans*-CH=CH-, *trans*-CF=CF-, -C=C- ou une liaison simple, et

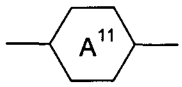
à
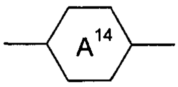
,

représentent, indépendamment les uns des autres,

a) 1,4-phénylène, où un ou plusieurs, de préférence un ou deux groupes CH peut/peuvent être remplacé(s) par N,
b) trans-1,4-cyclohexylène ou cyclohexénylène, où, en outre, un ou deux groupes $CH_2$ non adjacents peuvent chacun être remplacés par -O- et/ou -S- et où H peut être remplacé par F,

et où, dans les groupes a) et b),
un ou plusieurs atomes de H peut/peuvent également être remplacé(s) par Br, Cl, F, CN, -NCS, -SCN, $SF_5$, $C_1$-$C_{10}$ alkyle, $C_1$-$C_{10}$ alcoxy, un groupe $C_1$-$C_{10}$ alkyle ou alcoxy mono- ou polyfluoré ou un groupe $C_3$-$C_6$ cycloalkyle.

**7.** Milieu cristallin liquide selon la revendication 5 ou 6, **caractérisé en ce que** la concentration des composés de la formule I dans le milieu est dans la plage de 5% à 95% au total.

**8.** Utilisation des composés de la formule I selon une ou plusieurs des revendications 5 ou 1 à 4 dans un mélange de cristaux liquides.

**9.** Utilisation des composés de la formule I selon une ou plusieurs des revendications 5 ou 1 à 4 dans un composant pour une technologie hautes fréquences.

**10.** Procédé pour la préparation d'un milieu cristallin liquide selon une ou plusieurs des revendications 5 à 7, **caractérisé en ce qu'**un ou plusieurs composés de la formule I, comme indiqué selon la revendication 5, est/sont mélangé(s) avec un ou plusieurs composés choisis parmi les composés de la formule II, comme indiqué selon la revendication 6, et en option avec un ou plusieurs autres composés et en option avec un ou plusieurs additifs.

**11.** Composant pour une technologie hautes fréquences, **caractérisé en ce qu'**il comprend un milieu cristallin liquide selon une ou plusieurs des revendications 5 à 7.

**12.** Composant selon la revendication 11, **caractérisé en ce qu'**il s'agit d'un ou de plusieurs déphaseurs connectés fonctionnellement.

**13.** Utilisation d'un milieu cristallin liquide selon une ou plusieurs des revendications 5 à 7 dans un composant pour une technologie hautes fréquences.

**14.** Antenne groupe commandée en phase, **caractérisée en ce qu'**elle comprend un ou plusieurs composants selon une ou plusieurs des revendications 11 ou 12.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102004029429 A
- JP 2005120208 A
- EP 2073290 A1
- WO 2008021208 A2
- JP 2004082439 A
- WO 2008021208 A


### In der Beschreibung aufgeführte Nicht-Patentliteratur


- **A. GAEBLER ; A. MOESSINGER ; F. GOELDEN et al.** Liquid Crystal-Reconfigurable Antenna Concepts for Space Applications at Microwave and Millimeter Waves. *International Journal of Antennas and Propagation,* 2009, vol. 2009, 7
- **A. PENIRSCHKE ; S. MÜLLER ; P. SCHEELE ; C. WEIL ; M. WITTEK ; C. HOCK ; R. JAKOBY.** Cavity Perturbation Method for Characterization of Liquid Crystals up to 35GHz. *34th European Microwave Conference -Amsterdam,* 545-548
- **WU, S.-T. ; HSU, C.-S. ; SHYU, K.-F.** *Appl. Phys. Lett.,* 1999, vol. 74 (3), 344-346
- **GAUZA et al.** *Jap. J. Appl. Phys.,* 2004, vol. 11A, 7634-7638
- Merck Liquid Crystals, Physical Properties of Liquid Crystals. *Merck KGaA,* November 1997
- **A. PENIRSCHKE ; S. MÜLLER ; P. SCHEELE ; C. WEIL ; M. WITTEK ; C. HOCK ; R. JAKOBY.** Cavity Perturbation Method for Characterization of Liquid Crystals up to 35GHz. *34th European Microwave Conference - Amsterdam,* 545-548
- **A. GAEBLER ; F. GÖLDEN ; S. MÜLLER ; A. PENIRSCHKE ; R. JAKOBY.** Direct Simulation of Material Permittivites. *12MTC 2009 - International Instrumentation and Measurement Technology Conference, Singapur,* 2009, 463-467
- **A. PENIRSCHKE ; S. MÜLLER ; P. SCHEELE ; C. WEIL ; M. WITTEK ; C. HOCK ; R. JAKOBY.** Cavity Perturbation Method for Characterization of Liquid Crystals up to 35 GHz. *34th European Microwave Conference - Amsterdam,* 545-548